# EUROPEAN PATENT APPLICATION

(11) **EP 4 831 155 A1**
(43) Date of publication of application: **28.10.2026**
(21) Application number: 25382403.1
(22) Date of filing: 21.04.2025
(51) Int. Cl.: C07C 245/08, C07D 205/04, C07D 211/28, C07D 295/12, A61K 31/435, A61K 31/15, A61P 27/06

(54) **LIGHT-CONTROLLED BETA-ADRENERGIC COMPOUNDS**

(71) Applicant: Consejo Superior De Investigaciones Científicas, 28006 Madrid (ES)
(72) Inventor: LLEBARIA SOLDEVILA, Amadeo, E-08034 Barcelona (ES); ROVIRA ALGANS, Xavier, E-08034 Barcelona (ES); CATENA RUIZ, Juan Lorenzo, E-08034 Barcelona (ES); SERRA COMAS, Maria del Carmen, E-08034 Barcelona (ES); DURAN CORBERA, Anna, E-08034 Barcelona (ES)
(74) Representative: ABG Intellectual Property Law, S.L.

(57) **Abstract**

The present invention relates to a diazocompound of formula (I) or a salt, solvate or stereoisomer thereof, to a pharmaceutical composition comprising said compound and to its use as a medicament, especially in the treatment of ocular diseases.

## Description

### FIELD OF THE INVENTION

The invention belongs to the technical field of light-controlled compounds. More specifically, the invention belongs to the technical field of light-controlled beta-adrenergic compounds.

### BACKGROUND ART

Glaucoma is a complex and progressive eye disease characterized by damage to the optic nerve, often due to elevated intraocular pressure (IOP). This condition is one of the leading causes of blindness worldwide (Forrest et al. Glob Health Sci Pract. 28;11(1)). Current treatment options primarily aim to reduce IOP through medications, laser therapy, or surgery, yet many of these treatments have limitations in terms of efficacy, side effects, or patient compliance (Bedrood S. et al. Clin Ophthalmol. 17:3899-3913). Therefore, there remains a critical need for novel therapeutic approaches that can more effectively manage glaucoma while minimizing adverse effects (Arbabi, A., et al. Clinical and Experimental Optometry, 2021 105(2), 157-165).

While beta-blockers are effective in managing chronic glaucoma, their use requires careful consideration of potential systemic side effects, particularly in patients with respiratory or cardiac conditions (Sica AD et al. J Clin Hypertens. 3(3):175-182). The most common side effects of beta-blockers used in glaucoma treatment include mainly respiratory and cardiac systemic side effects, which require a fine control of the patient's medication. Another possible application of beta blockers in eye diseases include diabetic corneal sensory nerve dysfunction (Yuan X et al. Commun Biol. 8(1):481).

Photopharmacology is an emerging field that involves the use of photoactivable compounds to precisely control biological activity in living organisms through light exposure (Hüll K et al. Chem Rev. 118(21):10710-10747). One of the most promising advancements in this field is based on the use of azobenzene compounds that can undergo reversible photoisomerization changes upon exposure to light, enabling dynamic regulation of their biological properties (Kobauri P et al. Angew Chem Int Ed Engl. 24;62(30): e202300681). Photopharmacology provides significant advantages over traditional drugs by enabling precise temporal and spatial control over therapeutic effects. Unlike conventional drugs, which typically exert systemic effects, azobenzenes can be activated or deactivated through light exposure at specific times and locations, enhancing treatment precision and minimizing off-target effects. This control also allows for reduced dosages and fewer side effects by activating the drug only in targeted areas. Furthermore, drug photoswitches exhibit increased selectivity, as their molecular properties can be altered in response to light, improving their interaction with biological targets. The ability to adjust drug activity in real-time also facilitates personalized treatments, tailoring therapies to the individual needs of patients for more efficient and focused disease management.

Previous research demonstrated that the development of light-controllable beta blockers based on a photopharmacology approach is possible (Duran-Corbera A et al. J Med Chem. 13;63(15):8458-8470; Shi S et al. Biochem Pharmacol. 226:116396; Bosma R, et al. iScience. 25(9):104882). These drugs demonstrated pharmacological activity in cells, zebrafish larvae reducing the heart rate (Duran-Corbera A et al. Angew Chem Int Ed Engl. 61(30): e202203449), and rabbit reducing ocular hypertension (Shi S et al. Biochem Pharmacol. 226:116396), in a light dependent manner. These reported compounds demonstrated their photoswitchable activity using light wavelengths in the UV-violet range, which is not abundant in visible light and may cause phototoxicity. In addition, the molecules developed to date which are active only upon illumination exhibit thermal relaxation to the inactive state from hours to days, reducing the local selectivity and precision required to minimize side effects in other tissues when administered systemically (Li X et al. J. Clin. Transl. Ophthalmol. 2025, 3(1); Casson R. et al. Clinical & Experimental Ophtalmology, 2022)

### BRIEF DESCRIPTION OF THE INVENTION

This invention is related to the development of beta blockers which are photoswitchable with light above 405 nm, which is highly present in the spectrum of sunlight and artificial light sources from medical devices, and which have short thermal relaxation times, preferably less than 10 minutes. The photochemical and pharmacological properties of the asset within this invention are ideal for the development of eye disease therapies, as the drug rapidly returns to its most stable configuration when light is not present. This allows for increased localized precision in future treatments, reducing side effects in internal organs and distant tissues.

In summary, the use of drug photoswitches provides a novel and highly advantageous approach compared to traditional drugs. By offering precise control over the timing, location, and intensity of drug action, this technique not only enhances treatment efficacy but also reduces side effects and opens the door for more personalized, targeted therapies. These advantages make photopharmacology a promising tool for the future of medicine, particularly in treating complex and chronic diseases, more particularly ocular diseases such as glaucoma, ocular hypertension and diabetic corneal sensory nerve dysfunction, which currently do not have satisfactory treatments. Therefore, the development of novel molecules as reversible visible-light antagonists for the localized and temporally restricted activation of beta-adrenoceptors is essential. These molecules should respond to wavelengths suitable for intraocular pressure (IOP) regulation under visible light while remaining inactive in respiratory and cardiac tissues, thereby preventing adverse effects such as bradycardia, hypotension, and respiratory complications, which are frequently observed in glaucoma patients. In order to achieve local selectivity and precision thereby minimizing side effects in other tissues when administered systemically addition, drug photoswitches should preferably exhibit short thermal relaxation periods.

The inventors have developed a series of compounds useful in the treatment of diseases such as glaucoma, ocular hypertension and diabetic corneal sensory nerve dysfunction, which can be activated with light having a wavelength higher than 405 nm and exhibiting short thermal relaxation periods, preferably a thermal relaxation time of less than10 minutes.

As mentioned above, the compounds of the present invention are photoswitchable, meaning that isomerization from the thermostable *trans* to the *cis* configuration occurs when applying light above 405 nm. The compounds of the present invention can be back-isomerized to their thermally stable isomer when light is not present. In the context of the present invention, the term "thermal relaxation time" refers to the time that a compound needs to be back-isomerized to their thermally stable isomer.

Therefore, a first aspect of the present invention relates to a compound of formula I 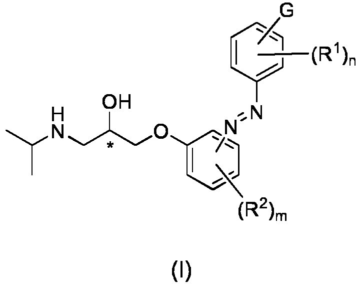
or a salt, solvate or stereoisomer thereof,
wherein
n is selected from 0, 1 and 2;
G is a nitrogen containing group selected from the group consisting of linear or branched amine and nitrogen containing heterocyclic ring, wherein said group is attached to the phenyl ring through a nitrogen atom;
   wherein the nitrogen containing heterocyclic ring is optionally substituted with a substituent selected from the group of linear or branched C₁₋₄ alkyl, C₁₋₄ alkoxy, OH, CN and halogen;
R¹ is selected from the group consisting of linear or branched C₁-C₆ alkyl optionally substituted with a OH group, OR³ group, halogen, OCF₃, CF₃, CN, NH₂, and NO₂;
   wherein R³ is selected from linear or branched C₁-C₃ alkyl;
m is selected from 0, 1 and 2; and
R² is selected from the group consisting of halogen, linear or branched C₁-C₆ alkyl optionally substituted with a OH group, OR⁴ group, halogen, OCF₃, CF₃, CN, NH₂, and NO₂;
   wherein R⁴ is selected from linear or branched C₁-C₃ alkyl.

A second aspect of the invention is related to a pharmaceutical composition comprising a compound of formula (I) as defined in the first aspect of the invention and at least one pharmaceutically acceptable excipient.

A third aspect of the invention is related to the compound of the first aspect of the invention for use as a medicament.

A further aspect of the invention relates to the compound of the first aspect of the invention for use in the treatment of an ocular disease.

### BRIEF DESCRIPTION OF FIGURES

**Figure 1****.** Depicts the maximum absorbance within the range of 300-600 nm of compounds of formula (I) in light and dark conditions.
**Figure 2****.** Depicts the thermal relaxation of some compounds of formula (I).
**Figure 3****.** Depicts the intraocular pressure at different times of pigmented C57BL6J mice treated with only 5% NaCl and treated with Timoftol^{®} (Gold Standard) applied topically immediately after NaCl injection.
**Figure 4****.** Depicts the intraocular pressure at different times of pigmented C57BL6J mice treated with 5% NaCl and I-17 (30 µM) under dark conditions and under light conditions.

### DETAILED DESCRIPTION OF THE INVENTION

A first aspect of the present invention relates to a compound of formula (I) 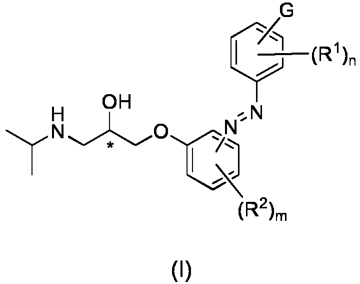
or a salt, solvate or stereoisomer thereof,
wherein
n is selected from 0, 1 and 2;
G is a nitrogen containing group selected from the group consisting of linear or branched amine and nitrogen containing heterocyclic ring, wherein said group is attached to the phenyl ring through a nitrogen atom;
wherein the nitrogen containing heterocyclic ring is optionally substituted with a substituent selected from the group of linear or branched C₁₋₄ alkyl, C₁₋₄ alkoxy, OH, CN and halogen.
R¹ is selected from the group consisting of linear or branched C₁-C₆ alkyl optionally substituted with a OH group, OR³ group, halogen, OCF₃, CF₃, CN, NH₂, and NO₂;
   wherein R³ is selected from linear or branched C₁-C₃ alkyl;
m is selected from 0, 1 and 2; and
R² is selected from the group consisting of halogen, linear or branched C₁-C₆ alkyl optionally substituted with a OH group, OR⁴ group, halogen, OCF₃, CF₃, CN, NH₂, and NO₂;
   wherein R⁴ is selected from linear or branched C₁-C₃ alkyl.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

In the context of the present invention, the term C₁-C₆ alkyl, refers to a linear or branched alkyl chain having from 1 to 6 carbon atoms. In an embodiment, of the present invention, alkyl groups contain preferably from 1 to 3 carbons atoms, more preferably from 1 to 2 atoms, even more preferably the alkyl group is a methyl group. Non-limiting examples of alkyl groups are: methyl, ethyl, propyl, isopropyl, butyl, isobutyl, *sec*-butyl, *tert*-butyl, pentyl, and hexyl.

In the context of the present invention, the term C₁-C₄ alkoxy, refers to a linear or branched alkyl chain having from 1 to 4 carbon atoms connected to an oxygen atom. In an embodiment, of the present invention, alkoxy groups contain preferably from 1 to 3 carbons atoms, more preferably from 1 to 2 atoms, even more preferably the alkoxy group is a methoxy group. Non-limiting examples of alkyl groups are: methoxy, ethoxy, propoxy, isopropoxy, butoxy, isobutoxy, *sec*-butoxy, *tert*-butoxy, pentoxy, and hexoxy.

In the context of the present invention the term "halogen" refers to F, CI, Br or I, preferably to Cl.

In the context of the present invention the term "nitrogen containing heterocyclic ring" refers to a cyclic group that comprises at least one atom of nitrogen on its ring. In the present invention the nitrogen containing heterocyclic ring are preferably saturated, meaning that all its bonds are simple bonds. The rings may comprise 1 to 2 substituents selected from the group consisting of C₁₋₄ alkyl, C₁₋₄ alkoxy, hydroxy, cyano and halogen. In an embodiment the rings have 3 to 7 members, preferably 3 to 6 members and comprise 1 to 2 heteroatoms selected from the group consisting of N, O and S, including at least 1 nitrogen atom. Examples of said nitrogen containing heterocyclic rings are piperidine, piperazine, N-methyl piperazine, morpholine, pyrrolidine, azetidine, aziridine, 1,3-diazetidine, pyrazolidine and imidazolidine.

When the compounds of the invention have chiral centers, they can exist in different stereoisomeric forms, such as enantiomeric or diastereomeric forms. As used herein, the term "stereoisomer" is a general term for all isomers of individual molecules that differ only in the orientation of their atoms in space, and include enantiomers and diastereomers. In this regard, the term "*" denotes the position of the molecule which constitutes the chiral centre.

Thus, any given compound referred to herein is intended to represent any one of a racemate, one or more enantiomeric forms and one or more diastereomeric forms. All the stereoisomers including enantiomers and diastereoisomers of the compounds referred to herein, and mixtures thereof (including racemic mixtures, enantiomerically enriched mixtures and diastereomerically enriched mixtures), are considered within the scope of the present invention.

The compounds described in the present description can be obtained as free compounds, as salts or as solvates (for example, hydrates, alcoholates, etc.), all forms being included within the scope of the present invention.

The term "salt" refers to a compound which is composed by two counterions, wherein one counterion is positively charged and the other counterion is negatively charged.

The term "solvate" refers to a complex formed by combining a compound of formula (I) or any other formula disclosed herein with a solvent, or an amount incorporated into a crystal structure. Solvation methods are generally known in the state of the art.

In the compound of formula (I), G is a nitrogen containing group selected from the group consisting of linear or branched amine and nitrogen containing heterocyclic ring, wherein said group is attached to the phenyl ring through a nitrogen atom.

In an embodiment, G is selected from the group consisting of N(C₁-C₆ alkyl)₂, NH₂, NH(C₁-C₆ alkyl), N(C₁-C₆ alkyl)(C₁-C₆ alkyl-OH) and nitrogen containing heterocyclic ring, preferably selected from the group consisting of N(C₁-C₃ alkyl)₂, NH₂; NH(C₁-C₃ alkyl), -N(C₁-C₃ alkyl)(C₁-C₃ alkyl-OH) and nitrogen containing heterocyclic ring, more preferably from the group consisting of N(C₁-C₂ alkyl)₂; NH₂; NH(C₁-C₂ alkyl) and N(C₁-C₂ alkyl)(C₁-C₂ alkyl-OH).

The C₁-C₆ alkyl, and the C₁-C₃ alkyl can be linear or branched alkyl groups.

As defined above, the nitrogen containing heterocyclic ring is optionally substituted with a substituent selected from the group of linear or branched C₁₋₄ alkyl, C₁₋₄ alkoxy, OH, CN and halogen.

In an embodiment, the nitrogen containing heterocyclic ring is selected from the group consisting of:

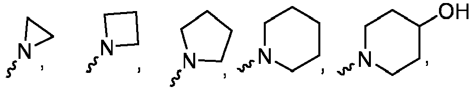

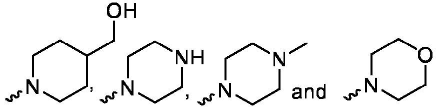

The G group can be located in any of the possible positions of the phenyl ring. In an embodiment, the G group is located in ortho or para position with respect to the diazo group, preferably in para position with respect to the diazo group.

In the context of the present invention, the term "diazo group" refers to the N=N group. In the present invention, the N=N group is linking two phenyl rings, which can be substituted.

In an embodiment, n is selected from 0, 1 and 2, preferably selected from 0 and 1.

When n is selected from 1 and 2, R¹ is selected from the group consisting of linear or branched C₁-C₆ alkyl optionally substituted with a OH group, OR³ group, halogen, OCF₃, CF₃, CN, NH₂, and NO₂. In an embodiment, R¹ is selected from linear or branched C₁-C₆ alkyl optionally substituted with a OH group and a OR³ group, preferably a linear or branched C₁-C₃ alkyl or a OCH₃ group.

As disclosed above, R³ is selected from linear or branched C₁-C₃ alkyl. In a preferred embodiment, R³ is a methyl group

The R¹ group can be located in any of the possible positions of the phenyl ring. In an embodiment, the R¹ group is located in ortho or meta position with respect to the diazo group, preferably in ortho.

In the compound of formula (I), m is selected from 0, 1 and 2, preferably from 0 and 1. In a preferred embodiment, m is 0.

When m is selected from 1 and 2, R² is selected from the group consisting of linear or branched C₁-C₆ alkyloptionally substituted with a OH group, OR⁴ group, halogen, OCF₃, CF₃, CN, NH₂, and NO₂, preferably from the group consisting of linear or branched C₁-C₆ alkyl optionally substituted with a OH group and a OR⁴ group, more preferably from linear or branched C₁-C₃ alkyl or a OCH₃ group.

As disclosed above, R⁴ is selected from linear or branched C₁-C₃ alkyl. In a preferred embodiment, R⁴ is a methyl group.

In an embodiment, the compound of formula (I) is characterized in that
n is selected from 0, 1 and 2;
G is selected from the group consisting of N(C₁-C₆ alkyl)₂, NH₂, NH(C₁-C₆ alkyl), N(C₁-C₆ alkyl)(C₁-C₆ alkyl-OH) and nitrogen containing heterocyclic ring;
   wherein the C₁-C₆ alkyl is selected from linear or branched C₁-C₆ alkyl optionally substituted;
   wherein the nitrogen containing heterocyclic ring is optionally substituted with a substituent selected from the group of linear or branched C₁₋₄ alkyl, C₁₋₄ alkoxy, OH, CN and halogen;
R¹ is selected from linear or branched C₁-C₆ alkyl optionally substituted with a OH group, and a OR³ group;
   wherein R³ is selected from linear or branched C₁-C₃ alkyl
m is selected from 0, 1 and 2; and
R² is selected from the group consisting of linear or branched C₁-C₆ alkyl optionally substituted with a OH group and a OR⁴ group;
   wherein R⁴ is selected from linear or branched C₁-C₃ alkyl.

In a preferred embodiment, the compound of formula (I) is characterized in that
n is selected from 0, and 1;
G is selected from the group consisting of N(C₁-C₃ alkyl)₂, NH₂; NH(C₁-C₃ alkyl), -N(C₁-C₃ alkyl)(C₁-C₃ alkyl-OH) and nitrogen containing heterocyclic ring;
   wherein the C₁-C₃ alkyl is selected from linear or branched C₁-C₃ alkyl optionally substituted;
   wherein the nitrogen containing heterocyclic ring is optionally substituted with a substituent selected from the group of linear or branched C₁₋₄ alkyl, C₁₋₄ alkoxy, OH, CN and halogen;
R¹ is linear or branched C₁-C₃ alkyl or a OCH₃ group;
m is selected from 0 and 1; and
R² is linear or branched C₁-C₃ alkyl or a OCH₃ group.

In an even more preferred embodiment, the compound of formula (I) is characterized in that
n is selected from 0, and 1;
G is selected from the group consisting of N(C₁-C₂ alkyl)₂; NH₂; NH(C₁-C₂ alkyl) and N(C₁-C₂ alkyl)(C₁-C₂ alkyl-OH);
   wherein the C₁-C₂ alkyl is selected from C₁-C₂ alkyl optionally substituted;
R¹ is linear or branched C₁-C₃ alkyl or a OCH₃ group;
m is selected from 0 and 1; and
R² is linear or branched C₁-C₃ alkyl or a OCH₃ group

The diazo group of the compound of formula (I) can be located in any of the position of the phenyl group with respect to the 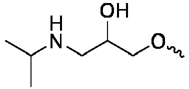 group, wherein the wavy line represents the point of attachment to the rest of the molecule.

In a preferred embodiment, the diazo group is located in meta position with respect to the 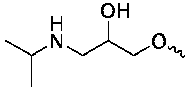 group.

In an embodiment, the compound of formula (I) is selected from the group consisting of:
*(S)*-1-(4-((4-(Dimethylamino)phenyl)diazenyl)phenoxy)-3-(isopropylamino) propan-2-ol,
*(R)*-1-(4-((4-(Dimethylamino)phenyl)diazenyl)phenoxy)-3-(isopropylamino) propan-2-ol,
1-(Isopropylamino)-3-(4-((4-(methylamino)phenyl)diazenyl)phenoxy)propan-2-ol,
1-(4-((4-(Azetidin-1-yl)phenyl)diazenyl)phenoxy)-3-(isopropylamino)propan-2-ol,
1-(Isopropylamino)-3-(4-((4-(pyrrolidin-1-yl)phenyl)diazenyl)phenoxy)propan-2-ol,
1-(Isopropylamino)-3-(4-((4-(piperidin-1-yl)phenyl)diazenyl)phenoxy)propan-2-ol,
1-(Isopropylamino)-3-(4-((4-morpholinophenyl)diazenyl)phenoxy)propan-2-ol,
1-(isopropylamino)-3-(4-((4-(4-methylpiperazin-1-yl)phenyl)diazenyl)phenoxy) propan-2-ol,
1-(4-((4-(Dimethylamino)-2-methoxyphenyl)diazenyl)phenoxy)-3-(isopropylamino)pr opan-2-ol,
1-(Isopropylamino)-3-(4-((2-methoxy-4-morpholinophenyl)diazenyl)phenoxy)propan-2-ol
1-(4-((2-(Dimethylamino)phenyl)diazenyl)phenoxy)-3-(isopropylamino)propan-2-ol,
1-(Isopropylamino)-3-(4-((2-(piperidin-1-yl)phenyl)diazenyl)phenoxy)propan-2-ol,
1-(Isopropylamino)-3-(4-((2-morpholinophenyl)diazenyl)phenoxy)propan-2-ol,
1-(4-((4-Aminophenyl)diazenyl)phenoxy)-3-(isopropylamino)propan-2-ol,
1-(4-((4-(Ethyl(2-hydroxyethyl)amino)phenyl)diazenyl)phenoxy)-3-(isopropylamino)pr opan-2-ol,
1-(4-((4-(Diethylamino)phenyl)diazenyl)phenoxy)-3-(isopropylamino)propan-2-ol,
(S)-1-(3-((4-(Dimethylamino)phenyl)diazenyl)phenoxy)-3-(isopropylamino)propan-2-ol,
1-(Isopropylamino)-3-(3-((4-(methylamino)phenyl)diazenyl)phenoxy)propan-2-ol,
1-(3-((4-(Azetidin-1-yl)phenyl)diazenyl)phenoxy)-3-(isopropylamino)propan-2-ol,
1-(Isopropylamino)-3-(3-((4-(pyrrolidin-1-yl)phenyl)diazenyl)phenoxy)propan-2-ol,
1-(Isopropylamino)-3-(3-((4-(piperidin-1-yl)phenyl)diazenyl)phenoxy)propan-2-ol,
1-(Isopropylamino)-3-(3-((4-morpholinophenyl)diazenyl)phenoxy)propan-2-ol,
1-(Isopropylamino)-3-(3-((4-(4-methylpiperazin-1-yl)phenyl)diazenyl)phenoxy) propan-2-ol,
1-(isopropylamino)-3-(3-((2-methoxy-4-morpholinophenyl)diazenyl)phenoxy) propan-2-ol,
1-(3-((2-(Dimethylamino)phenyl)diazenyl)phenoxy)-3-(isopropylamino)propan-2-ol,
1-(Isopropylamino)-3-(3-((2-(piperidin-1-yl)phenyl)diazenyl)phenoxy)propan-2-ol,
1-(Isopropylamino)-3-(3-((2-(morpholinophenyl)diazenyl)phenoxy)propan-2-ol
1-(3-((4-Aminophenyl)diazenyl)phenoxy)-3-(isopropylamino)propan-2-ol,
1-(3-((4-(Ethyl(2-hydroxyethyl)amino)phenyl)diazenyl)phenoxy)-3-(isopropylamino)pr opan-2-ol,
1-(3-((4-(Diethylamino)phenyl)diazenyl)phenoxy)-3-(isopropylamino)propan-2-ol,
*(S)*-1-(2-((4-(Dimethylamino)phenyl)diazenyl)phenoxy)-3-(isopropylamino)propan-2-ol,
1-(Isopropylamino)-3-(2-((4-(methylamino)phenyl)diazenyl)phenoxy)propan-2-ol,
1-(2-((4-(azetidin-1-yl)phenyl)diazenyl)phenoxy)-3-(isopropylamino)propan-2-ol,
1-(Isopropylamino)-3-(2-((4-(piperidin-1-yl)phenyl)diazenyl)phenoxy)propan-2-ol,
1-(Isopropylamino)-3-(2-((4-morpholinophenyl)diazenyl)phenoxy)propan-2-ol,
1-(isopropylamino)-3-(2-((4-(4-methylpiperazin-1-yl)phenyl)diazenyl)phenoxy) propan-2-ol,
1-(isopropylamino)-3-(2-((2-methoxy-4-morpholinophenyl)diazenyl)phenoxy)propan-2-ol,
1-(Isopropylamino)-3-(2-((2-(piperidin-1-yl)phenyl)diazenyl)phenoxy)propan-2-ol,
1-(2-((4-(Ethyl(2-hydroxyethyl)amino)phenyl)diazenyl)phenoxy)-3-(isopropylamino)pro pan-2-ol,
1-(2-((4-(Diethylamino)phenyl)diazenyl)phenoxy)-3-(isopropylamino)propan-2-ol, and
1-(Isopropylamino)-3-(2-((2-morpholinophenyl)diazenyl)phenoxy)propan-2-ol.

In a preferred embodiment, the compound of formula (I) is selected from the group consisting of
(S)-1-(3-((4-(Dimethylamino)phenyl)diazenyl)phenoxy)-3-(isopropylamino)propan-2-ol,
1-(Isopropylamino)-3-(3-((4-(methylamino)phenyl)diazenyl)phenoxy)propan-2-ol,
1-(3-((4-(Azetidin-1-yl)phenyl)diazenyl)phenoxy)-3-(isopropylamino)propan-2-ol,
1-(Isopropylamino)-3-(3-((4-(pyrrolidin-1-yl)phenyl)diazenyl)phenoxy)propan-2-ol,
1-(Isopropylamino)-3-(3-((4-(piperidin-1-yl)phenyl)diazenyl)phenoxy)propan-2-ol,
1-(Isopropylamino)-3-(3-((4-morpholinophenyl)diazenyl)phenoxy)propan-2-ol,
1-(Isopropylamino)-3-(3-((4-(4-methylpiperazin-1-yl)phenyl)diazenyl)phenoxy) propan-2-ol,
1-(isopropylamino)-3-(3-((2-methoxy-4-morpholinophenyl)diazenyl)phenoxy) propan-2-ol,
1-(3-((2-(Dimethylamino)phenyl)diazenyl)phenoxy)-3-(isopropylamino)propan-2-ol,
1-(Isopropylamino)-3-(3-((2-(piperidin-1-yl)phenyl)diazenyl)phenoxy)propan-2-ol,
1-(Isopropylamino)-3-(3-((2-morpholinophenyl)diazenyl)phenoxy)propan-2-ol,
1-(3-((4-Aminophenyl)diazenyl)phenoxy)-3-(isopropylamino)propan-2-ol,
1-(3-((4-(Ethyl(2-hydroxyethyl)amino)phenyl)diazenyl)phenoxy)-3-(isopropylamino)pr opan-2-ol, and
1-(3-((4-(Diethylamino)phenyl)diazenyl)phenoxy)-3-(isopropylamino)propan-2-ol.

In an even more preferred embodiment, the compound of formula (I) is selected from the group consisting of
(S)-1-(3-((4-(Dimethylamino)phenyl)diazenyl)phenoxy)-3-(isopropylamino)propan-2-ol,
1-(Isopropylamino)-3-(3-((4-(methylamino)phenyl)diazenyl)phenoxy)propan-2-ol, and
1-(3-((4-(Diethylamino)phenyl)diazenyl)phenoxy)-3-(isopropylamino)propan-2-ol.

A second aspect of the invention is related to a pharmaceutical composition comprising a compound of formula (I) as defined in any of the embodiments of the first aspect of the invention, or a salt, solvate or stereoisomer thereof, and at least one pharmaceutically acceptable excipient.

In the context of the present invention, the term "pharmaceutically acceptable excipient" refers to an excipient that is useful in preparing a pharmaceutical composition that is generally safe, non-toxic and neither biologically nor otherwise undesirable.

A third aspect of the invention is related with the compound of formula (I) according to any of its embodiments for use as a medicament.

A fourth aspect of the invention relates to the compound of the first aspect of the invention for use in the treatment of an ocular disease. In an embodiment, the ocular disease is selected from the group consisting of glaucoma, ocular hypertension and diabetic corneal sensory nerve dysfunction, preferably glaucoma.

Alternatively, the fifth aspect of the invention can be defined as a method of treatment or prevention of ocular disease, said method comprising administering and effective amount of a compound as defined in any of the embodiments of the first aspect of the invention to a subject in need thereof. In a preferred embodiment, the ocular disease is selected from the group consisting of glaucoma, ocular hypertension and diabetic corneal sensory nerve dysfunction, preferably glaucoma, even more preferably glaucoma.

The fourth aspect of the invention can be further defined as the use of a compound as defined in any of the embodiments of the first aspect of the invention for the preparation of a medicament for the treatment of prevention of ocular diseases. In a preferred embodiment, the ocular disease is selected from the group consisting of glaucoma, ocular hypertension and diabetic corneal sensory nerve dysfunction, preferably glaucoma.

Compounds of formula (I) may be synthesized by using some of the methods described below, as well as other processes known in the field of the organic chemistry. Preferred methods include, but are not limited to, the general procedures shown in the following Schemes.

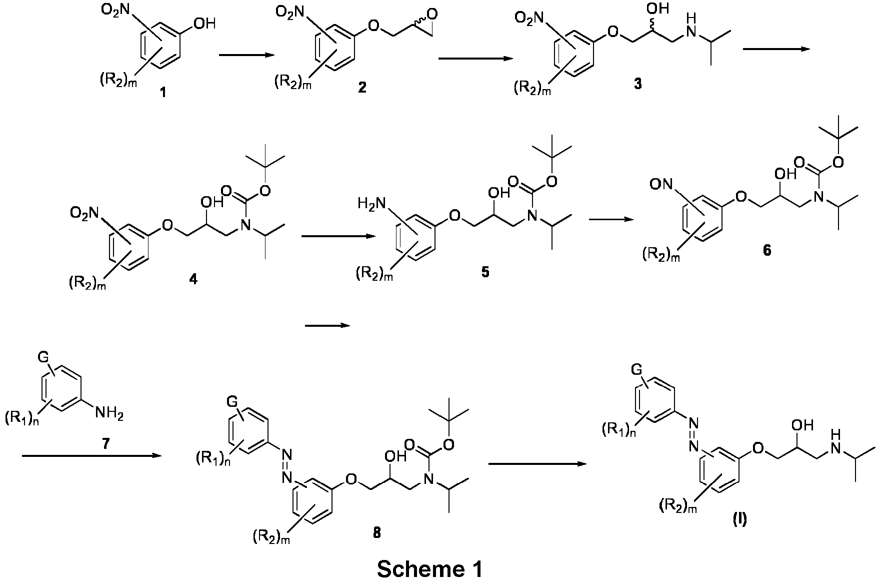

In general, the compound of formula (I) can be achieved by carbamate cleavage of intermediate **8,** which can be obtained by using Mills conditions between the nitroso intermediates type **6** and the amino intermediates type **7**.

Intermediates type **6** can be prepared from amino intermediates type **5** by oxidation according to procedures described in the literature such as reaction with Oxone^{®}.

Finally, the amino intermediate type **5** can be prepared by means of a Williamson reaction between phenol intermediates type **1** and a commercial glycidyltosilate or epichlorhidrine (*R, S* or racemic), obtaining the epoxide intermediates type **2,** which can be opened with commercial isopropylamine, obtaining the aminoalcohol intermediate **3**. Such compound was first protected with Boc anhydride (intermediate **4)** and then reduced to amine according to procedures described in the literature (as hydrogenation with Pd/C).

As alternative compound of formula (I) can be also prepared as is shown in scheme 2.

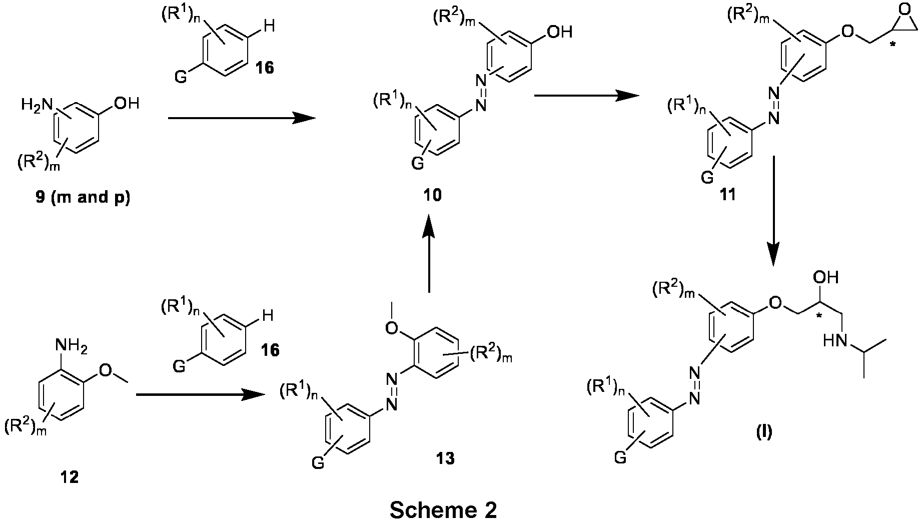

In this case, phenolic azobenzenes **10** (meta or para position) **are** obtained via azotization between the diazonium salt of the aminophenol **9** and anilines **16,** or via azotization between the diazonium salt of the aminoanisoles **12** and anilines **16** and subsequent demethylation of compound **13** (o position) with BBr₃. Then such compounds **10** can be alkylated with epichlorohydrin, and the isolated epoxides **11** were finally opened by nucleophilic attack of isopropylamine in order to obtain compound of formula (I).

As alternative compound (I) can be obtained from intermediate **10,** in a one pot reaction with Glycidyltosylate, instead of epichlorhidrine, and nucleophilic attack of isopropylamine.

On the other hand, intermediate **10** can be also prepared as is shown in Scheme 3. In this case, the phenolic intermediate **10** can be isolated from a typical Mills reaction, where nitrosoanilines **14** was condensed with aminophenol **9** or aminoanisole **12** and subsequent demethylation.

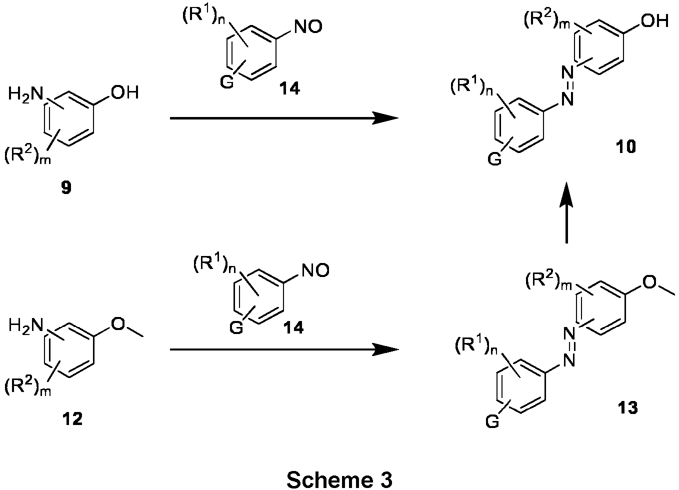

As alternative, intermediate 10 can be also prepared via diazotization reaction as is shown in Scheme 4.

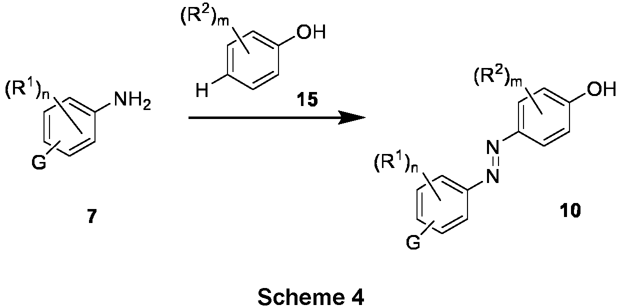

### Abbreviations

- Abs: Absorbance
- ACN: Acetonitrile
- AcOH: Acetic acid
- br: Broad
- brd: Broad doublet
- CV: Column Volume
- ESI: Electrospray lonisation
- EtOAc: Ethyl Acetate
- EtOH: Ethanol
- FIA: Flux Injected Analysis
- d: Doublet
- dd: Doublet of Doublets
- ddd: Doublet of Doublet of Doublets
- dddd: Doublet of Doublet of Doublet of Doublets
- DAD: Diode Array Detector
- DCM: Dichloromethane
- DIPEA: Diisopropylamine
- DME: Dimethoxiethane
- DMF: *N,N*-Dimethylformamide
- DMSO: Dimethylsulfoxide
- dt: Doublet of triplet
- ee: enantiomeric excess
- ESI: Electrospray lonization
- h: Hours
- HPLC: High-performance Liquid Chromatography
- IPA: Isopropanol
- LAH: Lithium aluminium hydride
- m: Multiplet
- MeOH: Methanol
- mg: Milligrams
- mL: Millilitre
- µL: Microliter
- MS: Mass Spectroscopy
- MHz: Mega Hertz
- min: Minutes
- mm: Millimetre
- mmol: Milimol
- m/z: Mass to Charge Ratio
- NMR: Nuclear Magnetic Resonance Spectrometry
- nm: Nanometre
- ppm: Parts Per Million
- q: Quartet
- r.t.: room temperature
- RT: Retention Time
- s: Singlet
- t: Triplet
- THF: Tetrahydrofuran
- UV: Ultraviolet

### Materials and methods

All the Chemicals and solvents are from commercial suppliers and used without purification, except the anhydrous solvents such as DMF which were treated previously through a system of solvent purification (PureSolv), degasified with inert gases and dried over alumina or molecular sieves.

Reactions were monitored by thin layer chromatography (60 F, 0.2 mm, Macherey-Nagel) by visualisation under 254 and/or 365 nm lamp. Purification was made by Flash column chromatography by using Merck Silica Gel 60, 40-63 microns RE or by Phase Reverse with an Isolera-Biotage equipment (SNAP KP-C18-HS; A: Water/Formic acid (0.05%), B: ACN/Formic Acid (0.05%): 5%B 3CV, 5%B-100%B 18CV, 100%B 5CV).

NMR were performed in a Brucker 400 MHz. Chemical shifts δ are reported in parts per million (ppm). Azo compounds existed in two isomeric forms, but in NMR spectra, only was assigned to the *trans* configuration based on abundance. HPLC 2795 Alliance Waters Aquity coupled to Detector DAD Agilent 1100 and Detector MS Waters ESI triple quadrupole Quattro micro, 10 µL of sample in ACN was injected, using a ZORBAX Extend-C18 3.5 µm 2.1x50mm (Agilent) column. The mobile phase used was a mixture of A = water + 0.05 formic acid and B = ACN + 0.05 formic acid with method described as follows: flow 0.5 mL/min; 5% B for 0.5 min; 5% to 100% B in 5 min, 100% B for 2 min.

Mass spectroscopy (MS) analysed by FIA with coupled to LCT Premier Orthogonal Accelerated Time of Flight Mass Spectrometer, acquiring data by ESI in positive mode. Spectra have been scanned between 50 and 1500 Da with values every 0.2 seconds and peaks are given m/z (% of basis peak).

UV analyses were performed at fixed wavelength (360 nm) for all compounds. The chiral analytical HPLC system consisted of a Dionex LPG-3400SD, equipped with a Dionex ACC-3000 autosampler, a Dionex VWD-3400-RS UV detector and collection data was performed using Chromeleon software from Thermofisher.

Enantiomeric purifications were conducted on a waters 1525P semi-prep HPLC coupled to a waters 2489 UV detector, a waters fraction collector III and collection data was performed using Empower software from Waters. Optical rotations were measured on a Perkin Elmer 341 polarimeter using a Na/Hal lamp set at 589 nm.

All compounds were synthesized following one of the general methods:

### General Procedure for Ether formation

Method A: To a stirred solution of intermediate type **1** or **10** (1 eq) in butanone (0.5 M), a base K₂CO₃ (3 eq) was added in small portions, and the reaction mixture was stirred at r.t. for 10 min. Then, epichlorohydrin (5 eq) (*R, S* or Racemic) was added, and the mixture was warmed to reflux overnight. The suspension was thereafter filtered and washed with acetone (x3); the red solution obtained was then evaporated in vacuo to afford the intermediate type **2** or **11.**

Method B: To a stirred solution of intermediate type **1** or **10** in anhydrous DMF (0.5 M). Potassium carbonate (1.2 eq) was then added to the solution and the mixture was left to stir under nitrogen for 10 minutes. (2*S*) or (2*R*)-Glycidyltosylate (1.2 eq) was then added and the reaction left to stir overnight at room temperature. Oxirane type **2** or **11** was not isolated and used directly on the subsequent step.

### General Procedure for epoxide-ring opening with isopropylamine.

Method C: intermediate type **2** or **11** (1 eq) was dissolved in isopropylamine (50 eq) and the reaction mixture was stirred at room temperature overnight. The reaction mixture was concentrated under reduced pressure, and the solid obtained was digested with diethyl-ether, yielding the intermediate type **3** as a solid, or was purified by column chromatography yielding the compounds of formula (I).

Method D: Oxirane type **2** or **11** formed via with Glycidyltosylate (Method B) was directly reacted with isopropylamine (20 eq) in a one-pot reaction and the mixture was heated up to 90°C for 48h. The reaction was then dried under vacuum and water was added. The obtained solution was neutralized using 2N NaOH and extracted using EtOAc. The organic fractions were poured together, dried over anhydrous Na₂SO₄, filtered and concentrated under reduced pressure. The product was further purified by reverse phase column, to give the amine-alcohol intermediate type 3, and the compounds of formula (I) as formate salts.

### General Procedure for protection of amines with BOC anhydride.

Method E: To a solution of amine-alcohol derivative type 3 (1 eq) in DCM (0.35 M) was added, dropwise, another solution of BOC-anhydride (1.05) in DCM (0.35 M) and stirred for 18 h. The reaction mixture was then evaporated under vacuum to afford carbamate compound type **4,** which was used without further purification.

### General Procedure for reduction of Nitrobenzenes to anilines.

Method F: To a solution of nitro derivative type **4** (1 eq) in MeOH (0.75 M) was added Pd-C (10% W/W) and the mixture was stirred for 18 h at rt under a hydrogen atmosphere. The reaction mixture was then filtered by using celite, and the filtrate was evaporated under vacuum and purified by flash chromatography (silica gel, EtOAc: hexane (1:2)) to afford pure amine compound type **5.**

### General Procedure for Nitrosation

Method G: Amino compound type **5** (1 eq) was dissolved in DCM-Water (1.5-2) (0.1 M) and OXONE (2 eq) were added. The reaction mixture was stirred vigorously at r.t. overnight. The mixture was extracted twice with DCM, the organic layer was dried over anhydrous MgSO₄, filtered and concentrated in vacuum. The residue was purified by flash chromatography (silica gel) to afford pure nitroso compounds type **6.**

### General Procedure for Azocompound formation.

Method H: Acid Mills reaction A solution of a nitroso compound type **6** or **14** (1.1 eq) and an amine type **7, 9** or **12** (1 eq) in AcOH [or a mixture of DCM-AcOH (1:2)] (0.1 to 0.01 M), was stirred at rt overnight. Then was concentrated under reduced pressure, and the residue was purified by reverse phase to provide the azocompound type **8, 10** or **13.**

Method I: Diazotization To an ice-cold solution of amine type **7, 9** or **12** (1eq) in HCl 2N (3 eq) and MeOH (0.4M) at 0°C was added, dropwise, sodium nitrite (1.2 eq) dissolved in water (0.4M) at 0 °C. The mixture was stirred for 30 min at 0°C and then it was added dropwise (always cold) to an ice-cold solution of aniline intermediate type **16** or phenol intermediate type **15** (1.2 eq) in aqueous NaOH or sodium acetate (1 M, 3 eq) and MeOH (0.1 M). The reaction mixture was kept and stirred at 0-5 °C. for 1h and left at rt for 2h. The precipitate formed was filtered off with cold water and dried to provide intermediate type **10** or **13.** In some cases, the residue was taken in EtOAc or DCM and water, and purified by column chromatography in silica, yielding the same intermediates type.

Method J: Basic Mills reaction A mixture of a nitroso compound type **6** or **14** (2 eq) an amine type **7, 9** or **12** (1 eq), and solid KOH (2.5 eq) was stirred at 90°C 10 min, then toluene (0.35 M) was added and the mixture was heated for an hour. After cooling down, the mixture was acidified to pH 7 using HCI 1M and extracted with EtOAc dried over Na₂SO₄, filtered and concentrated under reduced pressure to provide the azocompound type **8, 10** or **13.** Further purification was achieved through column chromatography in SiO₂

### General Procedure for anisole cleavage

Method K: Anisole derivatives type **13** (1 eq) was taken up in anhydrous DCM (60 0.1 M) and kept under a nitrogen atmosphere. The solution was cooled down to -78°C and a solution of 1M boron tribromide in DCM (1.5 eq) in DCM (60 mL) was added. After the addition, the reaction mixture was left to naturally warm up to room temperature and left to stir for 24h. Water was added to the reaction mixture and the two phases were extracted with DCM. The organic extracts were poured together, dried over Na₂SO₄, filtered and concentrated under reduced pressure to yield phenol derivatives type **10.**

### General Procedure for Boc cleavage.

Method L: Carbamate **8** (1 eq) was taken up in a mixture of DCM/TFA (0.1 M, 10:1) and TFA. The solution was stirred for 1h. Then the solution was concentrated under reduced pressure to yield compounds of formula (I) as trifluoroacetate salt.

### General Procedure for enantiomeric excess analysis of chiral compounds.

Method M: Enantiomeric excess was determined by Chiral analytical HPLC using the following binary systems in s chiral HPLC column, Phenomenex Lux Amylose-2 (4.6 x 250 mm, 5 µM): (S1) 0.25% IPA + 0,3 % DEA + 0,1 % HF in ACN, isocratic; 0.5 mL/min; (S2) 0.5% IPA + 0.1 % DEA + 0.1 % HF in ACN, isocratic; 0.6 mL/min; (S3) 1.5 % IPA + 0.1 % DEA in ACN, isocratic; 0.5 mL/min; (S4) 10 % IPA in Hexane + 0.1% DEA, isocratic; 1.0 mL/min; (S5) 5 % IPA in ACN + 0.1% DEA, isocratic; 0.5 mL/min.

The present invention will be described in further detail with reference to the following examples. These examples are for illustrative purposes only and are not intended to limit the scope of the present invention.

### EXAMPLES

### Intermediates

### 2-((2-Nitrophenoxy)methyl)oxirane (2o)

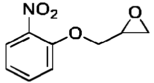

According to method A, from 3.00 g of 2-nitrophenol (64.7 mmol), 4.17 g of intermediate **(2o)** were obtained (99% yield) as a red solid.

¹H-NMR (400 MHz, CDCl₃) 7.87 (dd, *J* = 8.0, 1.6 Hz, 1H), 7.55 (ddd, J = 8.4, 7.6, 1.6 Hz, 1H), 7.15 (dd, *J* = 8.4, 1.1 Hz, 1H), 7.09 (ddd, *J* = 8.0, 7.6, 1.1 Hz, 1H), 4.43 (dd, *J =* 11.2, 2.9 Hz, 1H), 4.18 (dd, *J =* 11.2, 5.2 Hz, 1H), 3.42 (dddd, *J* = 5.2, 4.1, 2.9, 2.6 Hz, 1H), 2.95 (dd, *J* = 5.0, 4.1 Hz, 1H), 2.89 (dd, *J* = 5.0, 2.6 Hz, 1H).

### 2-((3-Nitrophenoxy)methyl)oxirane (2m)

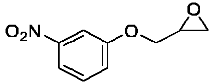

According to method A, from 3.73 g of 3-nitrophenol (57.6 mmol), 4.17 g of intermediate **(2m)** were obtained (99% yield) as an amber solid.

¹H-NMR (400 MHz, CDCl₃) δ 7.87 (ddd, *J* = 8.2, 2.4, 1.2 Hz, 1H), 7.78 (t, *J* = 2.4 Hz, 1H), 7.47 (t, *J* = 8.2 Hz, 1H), 7.30 (ddd, *J* = 8.2, 2.4, 1.2 Hz, 1H), 4.39 (dd, J = 11.1, 2.8 Hz, 1H), 4.02 (dd, J = 11.1, 5.9 Hz, 1H), 3.41 (dddd, J = 5.9, 4.1, 2.8, 2.6 Hz, 1H), 2.97 (dd, *J* = 4.8, 4.1 Hz, 1H), 2.82 (dd, *J* = 4.8, 2.6 Hz, 1H).

### 2-((4-Nitrophenoxy)methyl)oxirane (2p)

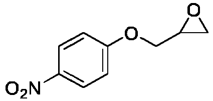

According to method A, from 2.90 g of 4-nitrophenol (62.5 mmol), 4.05 g of intermediate **(2p)** were obtained (99% yield) as an amber solid.

¹H-NMR (400 MHz, CDCl₃) δ **8.23** (d, *J* = 9.3 Hz, 2H), 7.02 (d, *J* = 9.3 Hz, 2H), 4.40 (dd, *J =* 11.1, 2.8 Hz, 1H), 4.04 (dd, *J =* 11.1, 5.8 Hz, 1H), 3.41 (dddd, *J* = 5.8, 4.1, 2.8, 2.6 Hz, 1H), 2.97 (dd, *J* = 4.8, 4.1 Hz, 1H), 2.81 (dd, *J* = 4.8, 2.6 Hz, 1H).

### 1-(Isopropylamino)-3-(2-nitrophenoxy)propan-2-ol (3o)

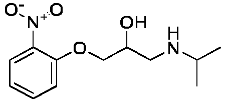

According to method C, from 4.09 g of intermediate **(2o)** (5.12 mmol), 5.17 g of intermediate **(3o)** were obtained (97% yield) as a white solid.

¹H-NMR (400 MHz, CDCl₃) δ 7.90 (dd, *J* = 8.4, 1.8 Hz, 1H), 7.56 (ddd, J = 8.4, 7.4, 1.8 Hz, 1H), 7.14 (dd, *J* = 8.4, 1.2 Hz, 1H), 7.07 (ddd, *J* = 8.4, 7.4, 1.2 Hz, 1H), 4.20 (dd, *J* = 9.3, 4.6 Hz, 1H), 4.16 (dd, *J =* 9.3, 5.1 Hz, 1H), 4.06 (dq, *J* = 6.4, 4.6 Hz, 1H), 2.93 (dd, *J* = 12.4, 4.6 Hz, 1H), 2.88-2.82 (m, 2H), 1.11 (dd, *J* = 6.3, 2.1 Hz, 6H).

### 1-(Isopropylamino)-3-(3-nitrophenoxy)propan-2-ol (3m)

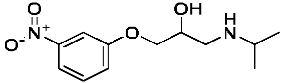

According to method C, from 1.00 g of intermediate **(2m)** (5.12 mmol), 910 mg of intermediate **(3m)** were obtained (70% yield) as a white solid.

¹H-NMR (400 MHz, CDCl₃) δ 7.86 (ddd, *J* = 8.2, 2.2, 1.0 Hz, 1H), 7.78 (t, *J* = 2.2 Hz, 1H), 7.46 (t, *J =* 8.2 Hz, 1H), 7.29 (ddd, *J* = 8.2, 2.2, 1.0 Hz, 1H), 4.10 - 4.00 (m, 3H), 2.93 (dd, *J* = 12.2, 3.8 Hz, 1H), 2.86 (p, *J* = 6.3 Hz, 1H), 2.75 (dd, *J* = 12.2, 7.6 Hz, 1H), 1.70-1.40 (brs, 2H), 1.12 (d, *J =* 6.3 Hz, 6H).

### 1-(Isopropylamino)-3-(4-nitrophenoxy)propan-2-ol (3p)

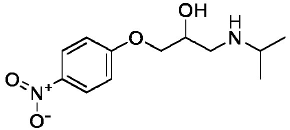

According to method C, from 1.00 g of intermediate **(2p)** (5.12 mmol), 1.02 g of intermediate **(3p)** were obtained (78% yield) as a white solid.

¹H-NMR (400 MHz, CDCl₃) δ 8.23 (d, *J* = 9.3 Hz, 2H), 7.01 (d, *J* = 9.3 Hz, 3H), 4.10 (d, *J* = 5.3 Hz, 2H), 4.07-4.00 (m, 1H), 2.93 (dd, *J* = 12.2, 3.9 Hz, 1H), 2.86 (p, *J =* 6.2 Hz, 1H), 2.74 (dd, *J* = 12.2, 7.9 Hz, 1H), 1.12 (d, *J* = 6.2 Hz, 6H).

### tert-Butyl (2-hydroxy-3-(2-nitrophenoxy)propyl)(isopropyl)carbamate (4o)

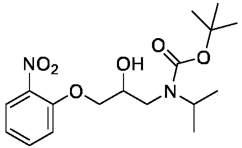

According to method E, from 5.17 g of intermediate **(3o)** (7.67 mmol), 7.21 g of intermediate **(4o)** were obtained (99% yield) as a white solid.

¹H-NMR (400 MHz, CDCl₃) δ 7.91 (dd, *J* = 7.8, 1.6 Hz, 1H), 7.56 (ddd, *J* = 8.4, 7.8, 1.6 Hz, 1H), 7.13 (d, *J =* 8.4 Hz, 1H), 7.08 (t, J = 7.8 Hz, 1H), 5.20 (br, 1H), 4.25-4.06 (m, 3H), 3.96 (br, 1H), 3.55 (d, *J* = 14.6 Hz, 1H), 3.42 (dd, *J* = 14.6, 8.0 Hz, 1H), 1.51 (s, 9H), 1.22 (d, *J* = 6.9 Hz, 3H), 1.19 (d, *J* = 6.7 Hz, 3H).

### tert-Butyl (2-hydroxy-3-(3-nitrophenoxy)propyl)(isopropyl)carbamate (4m)

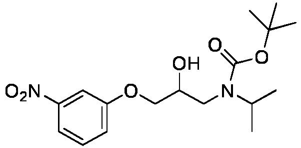

According to method E, from 2.97 g of intermediate **(3m)** (7.67 mmol), 4.10 g of intermediate **(4m)** were obtained (99% yield) as a white solid.

¹H-NMR (400 MHz, CDCl₃) δ 7.84 (ddd, *J* = 8.2, 2.4, 1.0 Hz, 1H), 7.76 (t, *J* = 2.4 Hz, 1H), 7.45 (t, *J* = 8.2 Hz, 1H), 7.26 (ddd, *J* = 8.2, 2.4, 1.0 Hz, 1H), 5.02 (br 1H), 4.25-4.03 (m, 3H), 4.02-3.94 (m, 1H), 3.50-3.41 (m, 1H), 3.38 (dd, *J* = 14.8, 2.9 Hz, 1H), 1.51 (s, 9H), 1.20 (d, *J* = 6.9 Hz, 3H), 1.16 (d, *J* = 6.7 Hz, 3H).

### tert-Butyl (2-hydroxy-3-(4-nitrophenoxy)propyl)(isopropyl)carbamate (4p)

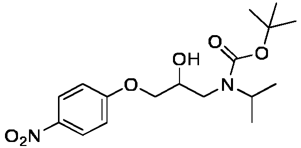

According to method E, from 1.95 g of intermediate **(3p)** (7.67 mmol), 2.72 g of intermediate **(4p)** were obtained (99% yield) as a white solid.

¹H-NMR (400 MHz, CDCl₃) δ 8.13 (d, *J* = 9.2 Hz, 3H), 6.91 (d, *J* = 9.2 Hz, 2H), 4.96 (br, 1H), 4.21-4.11 (m, 3H), 4.08-4.00 (m, 1H), 3.42-3.32 (m, 1H), 3.29 (dd, *J* = 14.9, 2.6 Hz, 1H), 1.42 (s, 9H), 1.10 (d, *J* = 6.9 Hz, 3H), 1.07 (d, *J* = 6.7 Hz, 3H).

### tert-Butyl (3-(2-aminophenoxy)-2-hydroxypropyl)(isopropyl)carbamate (5o)

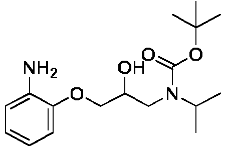

According to method F, from 7.21 g of intermediate **(4o)** (11.6 mmol), 5.57 g of intermediate **(5o)** were obtained (84% yield) as a pale-yellow oil.

¹H-NMR (400 MHz, CDCl₃) δ 6.87-6.80 (m, 2H), 6.78-6.71 (m, 2H), 4.94 (br, 1H), 4.15 (br, 2H), 4.04 (dd, *J* = 9.5, 5.0 Hz, 1H), 3.97 (br, 1H), 3.82 (br, 2H), 3.44 (br, 1H) (dd, *J* = 14.8, 3.2 Hz, 1H), 1.51 (s, 9H), 1.20 (d, *J* = 6.9 Hz, 3H), 1.16 (d, *J* = 6.7 Hz, 3H).

### tert-Butyl (3-(3-aminophenoxy)-2-hydroxypropyl)(isopropyl)carbamate (5m)

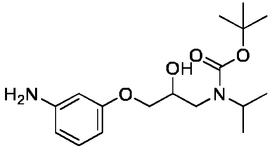

According to method F, from 4.10 g of intermediate **(4m)** (11.6 mmol), 1.85 g of intermediate **(5m)** were obtained (49% yield) as a pale-yellow oil.

¹H-NMR (400 MHz, CDCl₃) δ 7.07 (t, *J =* 8.0 Hz, 1H), 6.34 (ddd, *J* = 8.0, 2.4, 1.2 Hz, 1H), 6.32 (ddd, *J* = 8.0, 2.4, 1.2 Hz, 1H), ), 6.27 (t, *J* = 2.4 Hz, 1H), 4.91 (br, 1H), 4.14 (br, 1H), 4.06 (br, 1H), 3.99 (br, 1H), 3.84 (br, 1H), 3.68 (br, 2H), 3.40 (br, 2H), 1.51 (s, 9H), 1.20 (d, *J* = 6.9 Hz, 3H), 1.16 (d, *J* = 6.7 Hz, 3H).

### tert-Butyl (3-(4-aminophenoxy)-2-hydroxypropyl)(isopropyl)carbamate (5p)

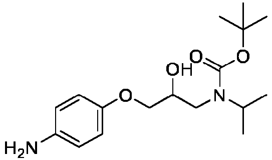

According to method F, from 2.75 g of intermediate **(4p)** (7.76 mmol), 0.95 g of intermediate **(5p)** were obtained (38% yield) as a pale-yellow oil.

¹H-NMR (400 MHz, CDCl₃) δ 6.77 (d, *J* = 8.8 Hz, 2H), 6.66 (d, *J* = 8.8 Hz, 2H), 4.84 (br, 1H), 4.05 (br, 1H), 3.96 (br, 1H), 3.82 (br, 1H), 3.45 br, 1H), 3.40 (br, 2H), 1.58 (br, 2H), 1.50 (s, 9H), 1.19 (d, *J* = 6.9 Hz, 3H), 1.16 (d, *J* = 6.7 Hz, 3H).

### tert-Butyl (2-hydroxy-3-(2-nitrosophenoxy)propyl)(isopropyl)carbamate (6o)

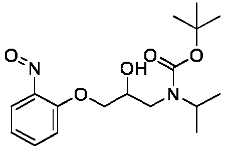

According to method G, from 5.57 g intermediate **(5o)** 17.2 mmol), 2.16 g of intermediate **(6o)** were obtained (37% yield) as a green oil, once it was purified by using EtOAc:hexane (1:3) as mobile phase.

¹H-NMR (400 MHz, CDCl₃) δ 7.59 (ddd, *J* = 8.4, 7.6, 1.8 Hz, 1H), 7.29 (d, *J =* 8.4 Hz, 1H), 6.82 (t, *J* = 7.6 Hz, 1H), 6.33 (dd, *J* = 7.6, 1.8 Hz, 1H), 5.15 (br, 1H), 4.40 (br, 1H), 4.23 (br, 2H), 4.05 (br, 1H), 3.60-3.49 (m, 1H), 3.48-3.36 (m, 1H), 1.42 (s, 9H), 1.08 (d, *J* = 6.9 Hz, 3H), 1.04 (d, *J* = 6.7 Hz, 3H).

### tert-Butyl (2-hydroxy-3-(3-nitrosophenoxy)propyl)(isopropyl)carbamate (6m)

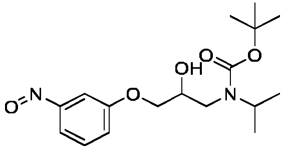

According to method G from 1.85 g intermediate **(5m)** 0.38 mmol), 792 mg of intermediate **(6m)** were obtained (41% yield) as an white solid once it was purified by using EtOAc:Hexane (1:3) as mobile phase.

¹H-NMR (400 MHz, CDCl₃) δ 7.94 (brd, *J* = 7.6 Hz, 1H), 7.54 (t, *J* = 8.2 Hz, 1H), 7.23 (ddd, *J* = 8.2, 2.7, 1.0 Hz, 1H), 6.88 (br, 1H), 4.89 (br, 1H), 4.15-3.95 (m, 3H), 3.87 (s, 1H), 3.44-3.28 (br, 1H), 3.29 (dd, *J* = 7.0, 2.1 Hz, 1H), 1.43 (s, 9H), 1.11 (d, *J =* 6.9 Hz, 3H), 1.07 (d, *J* = 6.7 Hz, 3H).

### tert-Butyl (2-hydroxy-3-(4-nitrosophenoxy)propyl)(isopropyl)carbamate (6p)

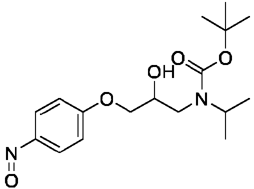

According to method G, from 950 mg of intermediate **(5p)** (0.38 mmol), 510 mg of intermediate **(6p)** were obtained (52% yield) as a green oil once it was purified by using EtOAc:hexane (1:3) as mobile phase.

¹H-NMR (400 MHz, CDCl₃) δ 7.94 (d, *J* = 8.8 Hz, 2H), 7.07 (d, *J* = 8.8 Hz, 2H), 5.06 (br, 1H), 4.22-4.09 (m, 3H), 4.08-3.99 (m, 1H), 3.52-3.42 (m, 1H), 3.39 (dd, *J* = 15.0, 2.7 Hz, 1H), 1.52 (s, 9H), 1.20 (d, *J* = 6.9 Hz, 3H), 1.16 (d, *J* = 6.7 Hz, 3H).

### tert-Butyl(3-(3-((4-(azetidin-1-yl)phenyl)diazenyl)phenoxy)-2-hydroxypropyl) (isopropyl)carbamate (8m-3)

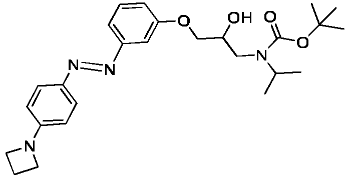

According to method H, from 22.5 mg of intermediate **(6m)** (0.07 mmol) and 4-(azetidin-1-yl)aniline, 10 mg of intermediate **(8m-3)** were obtained (32% yield) once it was purified by reverse phase chromatography.

HPLC-MS: retention time= 5.22 min; m/z: 469 [M+1]⁺.

### tert-Butyl2-hydroxy-3-(3-((4-(pyrrolidin-1-yl)phenyl)diazenyl)phenoxy)propyl) (isopropyl)carbamate (8m-4)

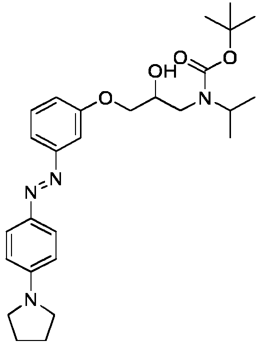

According to method H, from 22.5 mg of intermediate (**6m)** (0.07 mmol) and 4-(pyrrolidin-1-yl)aniline, 10 mg of intermediate (**8m-4**) were obtained (26% yield) once it was purified by reverse phase chromatography.

HPLC-MS: retention time= 5.24 min; m/z: 483 [M+1]⁺.

### tert-Butyl(2-hydroxy-3-(3-((4-(piperidin-1-yl)phenyl)diazenyl)phenoxy)propyl) (isopropyl)carbamate (8m-5)

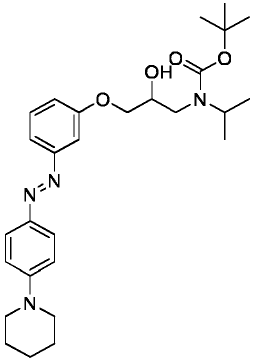

According to method H, from 22.5 mg of intermediate **(6m)** (0.07 mmol) and 4-(piperidin-1-yl)aniline, 2.17 mg of intermediate (8m-5) were obtained (6% yield) once it was purified by reverse phase chromatography.

HPLC-MS: retention time= 5.70 min; m/z: 497 [M+1]⁺.

### tert-Butyl(2-hydroxy-3-(3-((4-morpholinophenyl)diazenyl)phenoxy)propyl) (isopropyl)carbamate (8m-6)

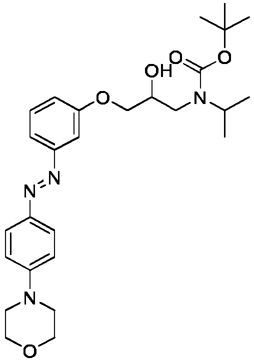

According to method H, from 22.5 mg of intermediate (**6m**) (0.07 mmol) and 4-morpholinoaniline, 10 mg of intermediate (**8m-6**) were obtained (36% yield) once it was purified by reverse phase chromatography.

HPLC-MS: retention time= 4.76 min; m/z: 499 [M+1]⁺.

### 4-((2-Methoxyphenyl)diazenyl)-N,N-dimethylaniline (13o)

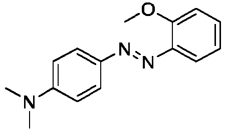

According to method H, from 2.15 g of 2-methoxyaniline (16.2 mmol) and 1-methoxy-2-nitrosobenzene, 3.9 g of intermediate **(13o)** were obtained (96% yield) as a red oil.

¹H-NMR (400 MHz, CDCl₃) δ 7.82 (d, *J =* 9.1 Hz, 2H), 7.54 (dd, *J* = 8.2, 1.7 Hz, 1H), 7.37 (ddd, *J* = 8.2, 7.2, 1.7 Hz, 1H), 7.16 (dd, *J* = 8.2, 1.1 Hz, 1H), 6.99 (ddd, *J* = 8.2, 7.2, 1.1 Hz, 1H), 6.81 (d, *J =* 9.1 Hz, 1H), 3.98 (s, 3H), 3.08 (s, 6H).

### 2-((4-(Dimethylamino)phenyl)diazenyl)phenol (10o)

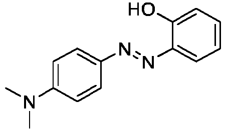

According to method J, from 1.3 g *N,N*-dimethyl-4-nitrosoaniline and 2-aminophenol, 196 mg of intermediate **(10o)** were obtained (9% yield) as an orange solid, once it was purified by using EtOAc:Hexane (1:4) as mobile phase.

According to method K, from 4 g of intermediate **(13o)** (15.7 mmol), 3.7 of intermediate **(10o)** were obtained (98% yield) as a brown solid.

¹H-NMR (400 MHz, CDCl₃) δ 7.82 (d, *J =* 9.1 Hz, 2H), 7.54 (dd, *J* = 8.2, 1.7 Hz, 1H), 7.37 (ddd, *J* = 8.2, 7.2, 1.7 Hz, 1H), 7.16 (dd, *J* = 8.2, 1.1 Hz, 1H), 6.99 (ddd, *J* = 8.2, 7.2, 1.1 Hz, 1H), 6.81 (d, *J* = 9.1 Hz, 1H), 3.98 (s, 3H), 3.08 (s, 6H).

### 3-((4-(Dimethylamino)phenyl)diazenyl)phenol (10m)

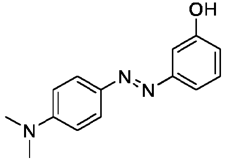

According to method I, from 2.15 g of 3-aminophenol (19.7 mmol) and N,N-dimethylaniline, 600 mg of intermediate (10m) were obtained (13% yield) as a brown oil, once it was purified by using EtOAc: Hexane (1:4) as mobile phase.

¹H-NMR (400 MHz, CDCl₃) δ 7.88 (d, *J* = 8.8 Hz, 2H), 7.46 (ddd, *J* = 7.9, 2.1, 1.0 Hz, 1H), 7.35 (t, *J* = 7.9 Hz, 1H), 7.31 (t, *J* = 2.1 Hz, 1H), 6.88 (ddd, *J* = 7.9, 2.1, 1.0 Hz, 1H), 6.76 (d, *J =* 8.8 Hz, 1H), 3.10 (s, 6H).

### 4-((4-(Dimethylamino)phenyl)diazenyl)phenol (10p)

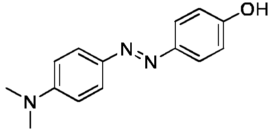

According to method I, from 2.15 g of commercial 4-aminophenol (19.7 mmol) and *N,N*-dimethylaniline, 3.17 g of intermediate **(10p)** were obtained (67% yield) as a red solid.

¹H-NMR (400 MHz, CDCl₃) δ 7.81 (d, *J* = 8.8 Hz, 2H), 7.74 (d, *J* = 8.8 Hz, 2H), 6.92 (d, *J* = 8.8 Hz, 2H), 6.74 (d, *J* = 8.8 Hz, 2H), 3.05 (s, 6H).

### (S)-N,N-Dimethyl-4-((2-(oxiran-2-ylmethoxy)phenyl)diazenyl)aniline ((S)-11o)

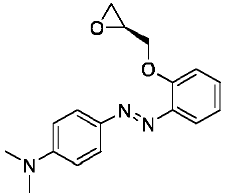

According to method A, from 560 mg of intermediate **(10o)** (0.2 mmol) and *R-*epichlorohydrin, 370 mg of intermediate **((*S*)-11o))** were obtained (91% yield) as an orange oil.

According to method B, from 1.15 g of intermediate **(10o)** (0.2 mmol) and (2*S*)-glycidyltosylate, 1.52 g of intermediate **((*S*)-11o))** were obtained (100% yield) as a brown oil.

¹H-NMR (400 MHz, CDCl₃) **δ** 7.90 (d, *J* = 9.0 Hz, 1H), 7.65 (dd, *J* = 8.0, 1.7 Hz, 1H), 7.33 (ddd, *J =* 8.3, 7.3, 1.7 Hz, 1H), 7.16-7.01 (m, 2H), 6.80 (d, *J* = 9.0 Hz, 2H), 4.42 (dd, *J =* 11.3, 3.4 Hz, 1H), 4.24 (dd, *J =* 11.3, 5.2 Hz, 1H), 3.50-3.42 (m, 1H), 3.10 (s, 6H), 2.97-2.89 (m, 1H), 2.85 (dd, *J =* 5.0, 2.6 Hz, 1H).

[α]^{25D}= - 1.0 (c = 1.0, MeOH).

### (S)-N,N-Dimethyl-4-((3-(oxiran-2-ylmethoxy)phenyl)diazenyl)aniline ((S)-11m)

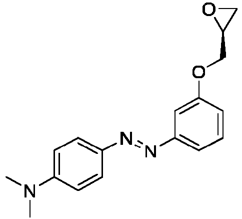

According to method A, from 330 mg of intermediate (**10m**) (0.2 mmol) and *R-*epichlorohydrin, 370 mg of intermediate **((*S*)-11o))** were obtained (91% yield) as an orange oil, once it was purified by column chromatography (6:1 Hex:EtOAc).

¹H-NMR (400 MHz, CDCl₃) δ 7.89 (d, *J* = 8.9 Hz, 2H), 7.50 (dt, *J* = 8.0, 1.3 Hz, 1H), 7.43-7.35 (m, 2H), 6.98 (ddd, *J* = 8.0, 2.6, 1.0 Hz, 1H), 6.77 (d, *J* = 8.9 Hz, 2H), 4.31 (dd, *J* = 10.9, 3.3 Hz, 1H), 4.07 (dd, *J* = 10.9, 5.6 Hz, 1H), 3.44-3.36 (m, 1H), 3.10 (s, 6H), 2.93 (dd, *J* = 4.9, 4.1 Hz, 1H), 2.80 (dd, *J* = 4.9, 2.6 Hz, 1H).

[α]^{25D}= + 13.4 (c = 1.0, MeOH).

### (S)-N,N-Dimethyl-4-((4-(oxiran-2-ylmethoxy)phenyl)diazenyl)aniline ((S)-11p)

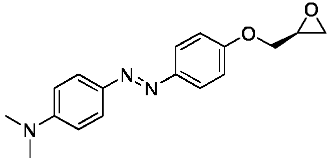

According to method A, from 65 mg of intermediate **(10p)** (0.2 mmol) and R-epichlorohydrin, 330 mg of intermediate **((*S*)-11p))** were obtained (99% yield) as a brown solid.

¹H-NMR (400 MHz, CDCl₃) δ 7.88-7.82 (m, 4H), 7.00 (d, *J* = 9.0 Hz, 2H), 6.77 (d, *J*= 8.8 Hz, 2H), 4.29 (dd, *J* = 11.0, 3.2 Hz, 1H), 4.03 (dd, *J* = 11.0, 5.6 Hz, 1H), 3.37 (dddd, *J =* 5.6, 4.1, 3.2, 2.6 Hz, 1H), 3.08 (s, 6H), 2.93 (dd, *J =* 4.9, 4.1 Hz, 1H), 2.79 (dd, *J* = 4.9, 2.6 Hz, 1H).

[α]^{25D}= + 8.4 (c = 1.0, MeOH).

### (R)-N,N-Dimethyl-4-((4-(oxiran-2-ylmethoxy)phenyl)diazenyl)aniline ((R)-11p)

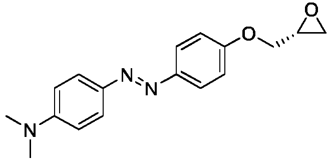

According to method A, from 800 mg of intermediate **(10p)** (3.32 mmol) and *S-*epichlorohydrin, 951 mg of intermediate **((*R*)-11p))** were obtained (99% yield) as a brown solid.

### tert-Butyl(2-hydroxy-3-(4-((4-(methylamino)phenyl)diazenyl)phenoxy)propyl) (isopropyl)carbamate (8p-2)

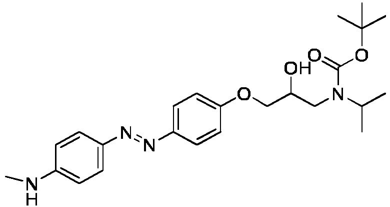

According to method H, from 28 mg of intermediate **(6p)** (0.08 mmol) and *N-*methylaniline, 2.6 mg of intermediate **(8p-2)** were obtained (7% yield) once it was purified by reverse phase chromatography.

HPLC-MS: retention time= 5.16 min; m/z: 443 [M+1]⁺.

### tert-Butyl(3-(4-((4-(azetidin-1-yl)phenyl)diazenyl)phenoxy)-2-hydroxypropyl) (isopropyl)carbamate (8p-3)

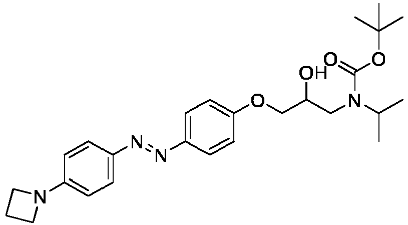

According to method H, from 28 mg of intermediate (**6p)** (0.08 mmol) and 4-(azetidin-1-yl)aniline, 4.8 mg of intermediate (**8p-3**) were obtained (12% yield) once it was purified by reverse phase chromatography.

HPLC-MS: retention time= 5.16 min; m/z: 469 [M+1]⁺.

### tert-Butyl2-hydroxy-3-(4-((4-(pyrrolidin-1-yl)phenyl)diazenyl)phenoxy)propyl)(i sopropyl)carbamate (8p-4)

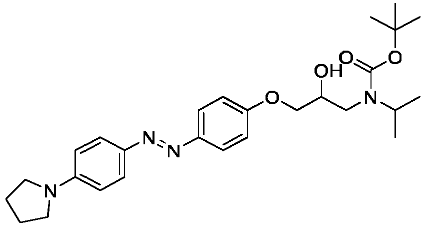

According to method H, from 28 mg of intermediate (**6p**) (0.08 mmol) and 4-(pyrrolidin-1-yl)aniline, 4.1 mg of intermediate **(8p-4)** were obtained (19% yield) once it was purified by reverse phase chromatography.

HPLC-MS: retention time= 5.46 min; m/z: 483 [M+1]⁺.

### tert-Butyl(2-hydroxy-3-(4-((4-(piperidin-1-yl)phenyl)diazenyl)phenoxy)propyl) (isopropyl)carbamate (8p-5)

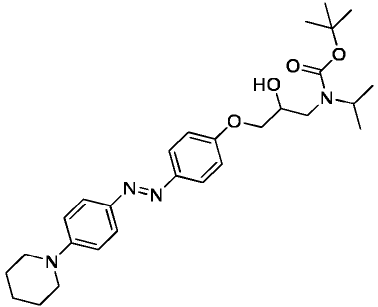

According to method H, from 10 mg of intermediate **(6p)** (0.03 mmol) and 4-(piperidin-1-yl)aniline, 4.1 mg of intermediate **(8p-5)** were obtained (27% yield) once it was purified by reverse phase chromatography.

HPLC-MS: retention time= 5.53 min; m/z: 497 [M+1]⁺.

### tert-Butyl(2-hydroxy-3-(4-((4-morpholinophenyl)diazenyl)phenoxy)propyl)(isop ropyl)carbamate (8p-6)

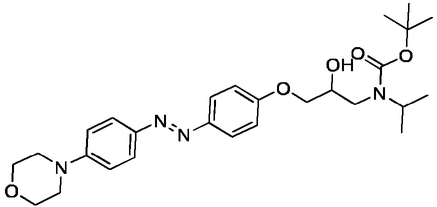

According to method H, from 28 mg of intermediate (**6p**) (0.08 mmol) and 4-morpholinoaniline, 4.0 mg of intermediate (**8p-6**) were obtained (10% yield) once it was purified by reverse phase chromatography.

HPLC-MS: retention time= 4.78 min; m/z: 499 [M+1]⁺.

### tert-Butyl(2-hydroxy-3-(4-((4-(4-methylpiperazin-1-yl)phenyl)diazenyl)phenoxy) propyl)(isopropyl)carbamate (8p-7)

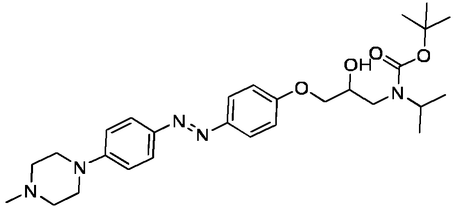

According to method H, from 28 mg of intermediate **(6p)** (0.08 mmol) and 4-(4-methylpiperazin-1-yl)aniline, 1.5 mg of intermediate **(8p-7)** were obtained (4% yield) once it was purified by reverse phase chromatography.

HPLC-MS: retention time= 3.20 min; m/z: 512 [M+1]⁺.

### tert-Butyl(3-(4-((4-(dimethylamino)-2-methoxyphenyl)diazenyl)phenoxy)-2-hydroxypropyl)(isopropyl)carbamate (8p-8)

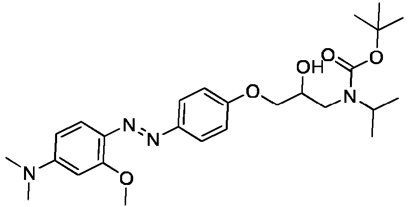

According to method H, from 28 mg of intermediate **(6p)** (0.08 mmol) and 3-methoxy-*N*1,*N*1-dimethylbenzene-1,4-diamine, 6.6 mg of intermediate **(8p-8)** were obtained (16% yield) once it was purified by reverse phase chromatography.

HPLC-MS: retention time= 4.18 min; m/z: 487 [M+1]⁺.

### tert-Butyl (2-hydroxy-3-(4-((2-methoxy-4-morpholinophenyl)diazenyl)phenoxy) propyl)(isopropyl)carbamate (8p-9)

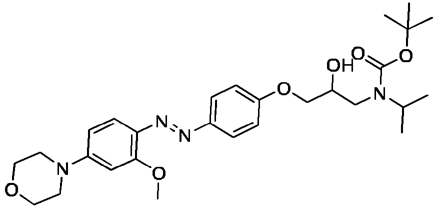

According to method H, from 28 mg of intermediate **(6p)** (0.08 mmol) and 2-methoxy-4-morpholinoaniline, 5.7 mg of intermediate **(8p-9)** were obtained (14% yield) once it was purified by reverse phase chromatography.

HPLC-MS: retention time= 4.47 min; m/z: 529 [M+1]⁺.

### tert-Butyl (3-(4-((2-(dimethylamino)phenyl)diazenyl)phenoxy)-2-hydroxypropyl) (isopropyl)carbamate (8p-10)

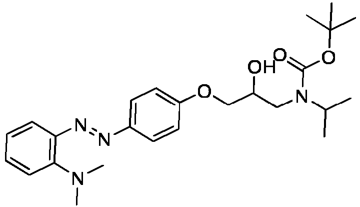

According to method H, from 28 mg of intermediate **(6p)** (0.08 mmol) and *N1,N1-*dimethylbenzene-1,2-diamine, 1.7 mg of intermediate **(8p-10)** were obtained (5% yield) once it was purified by reverse phase chromatography.

HPLC-MS: retention time= 4.09 min; m/z: 457 [M+1]⁺.

### tert-Butyl (2-hydroxy-3-(4-((2-(piperidin-1-yl)phenyl)diazenyl)phenoxy)propyl) (isopropyl)carbamate (8p-11)

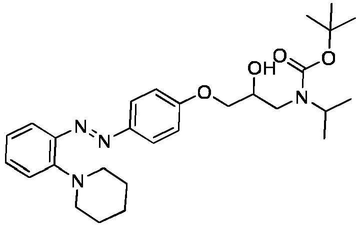

According to method H, from 28 mg of intermediate **(6p)** (0.08 mmol) and 2-(piperidin-1-yl)aniline, 7.2 mg of intermediate **(8p-11)** were obtained (18% yield) once it was purified by reverse phase chromatography.

HPLC-MS: retention time= 4.54 min; m/z: 497 [M+1]⁺.

### tert-Butyl(2-hydroxy-3-(4-((2-morpholinophenyl)diazenyl)phenoxy)propyl) (isopropyl)carbamate (8p-12)

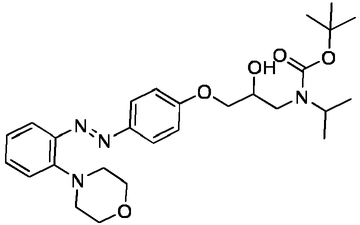

According to method H, from 28 mg of intermediate **(6p)** (0.08 mmol) and 2-morpholinoaniline, 5.9 mg of intermediate **(8p-12)** were obtained (14% yield) once it was purified by reverse phase chromatography.

HPLC-MS: retention time= 4.79 min; m/z: 499 [M+1]⁺.

### tert-Butyl(2-hydroxy-3-(4-((4-(amino)phenyl)diazenyl)phenoxy)propyl)(isopropy I)carbamate (8p-13)

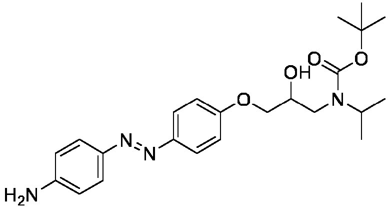

According to method H, from 28 mg of intermediate (**6p)** (0.08 mmol) and benzene-1,4-diamine, 4.0 mg of intermediate (**8p-2**) were obtained (12% yield) once it was purified by reverse phase chromatography.

HPLC-MS: retention time= 4.21 min; m/z: 429 [M+1]⁺.

### tert-Butyl(3-(4-((4-(ethyl(2-hydroxyethyl)amino)phenyl)diazenyl)phenoxy)-2-hydroxypropyl)(isopropyl)carbamate (8p-14)

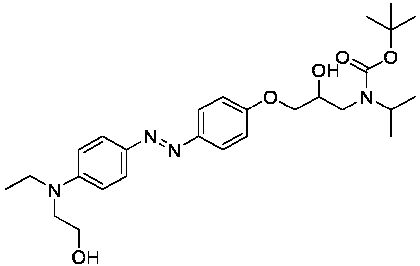

According to method H, from 28 mg of intermediate (**6p**) (0.08 mmol) and 2-((4-aminophenyl)(ethyl)amino)ethan-1-ol, 4.2 mg of intermediate **(8p-14)** were obtained (10% yield) once it was purified by reverse phase chromatography.

HPLC-MS: retention time= 4.43 min; m/z: 501 [M+1]⁺.

### tert-Butyl(3-(4-((4-(diethylamino)phenyl)diazenyl)phenoxy)-2-hydroxypropyl) (isopropyl)carbamate (8p-15)

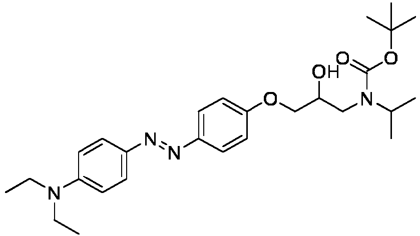

According to method H, from 28 mg of intermediate **(6p)** (0.08 mmol) and *N1,N1-*diethylbenzene-1,4-diamine, 3.9 mg of intermediate **(8p-15)** were obtained (10% yield) once it was purified by reverse phase chromatography.

HPLC-MS: retention time= 5.37 min; m/z: 485 [M+1]⁺.

### tert-Butyl(2-hydroxy-3-(3-((4-(methylamino)phenyl)diazenyl)phenoxy)propyl) (isopropyl)carbamate (8m-2)

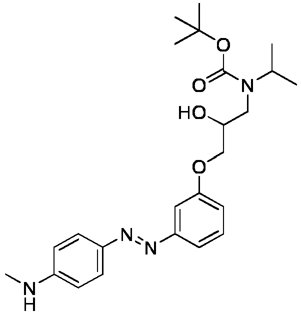

According to method H, from 28 mg of intermediate **(6m)** (0.08 mmol) and *N-*methylaniline, 3.3 mg of intermediate **(8m-2)** were obtained (9% yield) once it was purified by reverse phase chromatography.

HPLC-MS: retention time= 4.60 min; m/z: 443 [M+1]⁺.

### tert-Butyl(3-(3-((4-(azetidin-1-yl)phenyl)diazenyl)phenoxy)-2-hydroxypropyl) (isopropyl)carbamate (8m-3)

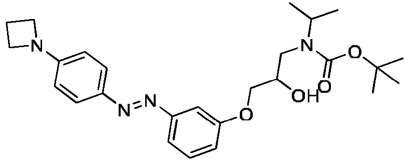

According to method H, from 22.5 mg of intermediate **(6m)** (0.07 mmol) and 4-(azetidin-1-yl)aniline, 12.3 mg of intermediate **(8m-3)** were obtained (39% yield) once it was purified by reverse phase chromatography.

HPLC-MS: retention time= 5.22 min; m/z: 469 [M+1]⁺.

### tert-Butyl2-hydroxy-3-(3-((4-(pyrrolidin-1-yl)phenyl)diazenyl)phenoxy)propyl)(i sopropyl)carbamate (8m-4)

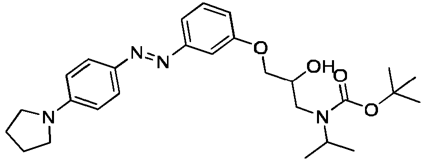

According to method H, from 22.5 mg of intermediate **(6m)** (0.07 mmol) and 4-(pyrrolidin-1-yl)aniline, 11.1 mg of intermediate **(8m-4)** were obtained (42% yield) once it was purified by reverse phase chromatography.

HPLC-MS: retention time= 5.42 min; m/z: 483 [M+1]⁺.

### tert-Butyl(2-hydroxy-3-(3-((4-(piperidin-1-yl)phenyl)diazenyl)phenoxy)propyl) (isopropyl)carbamate (8m-5)

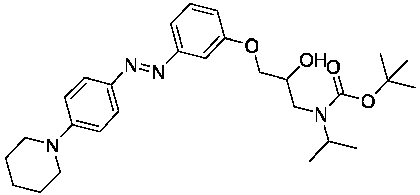

According to method H, from 22.5 mg of intermediate **(6m)** (0.07 mmol) and 4-(piperidin-1-yl)aniline, 12.2 mg of intermediate **(8m-5)** were obtained (44% yield) once it was purified by reverse phase chromatography.

HPLC-MS: retention time= 5.60 min; m/z: 497 [M+1]⁺.

### tert-Butyl(2-hydroxy-3-(3-((4-morpholinophenyl)diazenyl)phenoxy)propyl) (isopropyl)carbamate (8m-6)

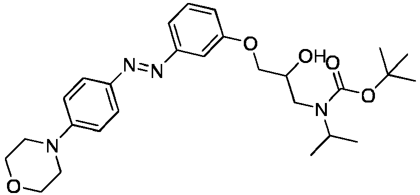

According to method H, from 22.5 mg of intermediate **(6m)** (0.13 mmol) and 4-morpholinoaniline, 13.1 mg of intermediate **(8m-6)** were obtained (47% yield) once it was purified by reverse phase chromatography.

HPLC-MS: retention time= 5.70 min; m/z: 499 [M+1]⁺.

### tert-Butyl(2-hydroxy-3-(3-((4-(4-methylpiperazin-1-yl)phenyl)diazenyl)phenoxy) propyl)(isopropyl)carbamate (8m-7)

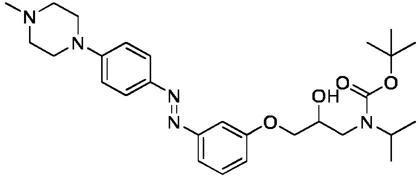

According to method H, from 28 mg of intermediate **(6m)** (0.08 mmol) and 4-(4-methylpiperazin-1-yl)aniline, 12.0 mg of intermediate **(8m-7)** were obtained (28% yield) once it was purified by reverse phase chromatography.

HPLC-MS: retention time= 3.09 min; m/z: 512 [M+1]⁺.

### tert-Butyl(2-hydroxy-3-(3-((2-methoxy-4-morpholinophenyl)diazenyl)phenoxy) propyl)(isopropyl)carbamate (8m-9)

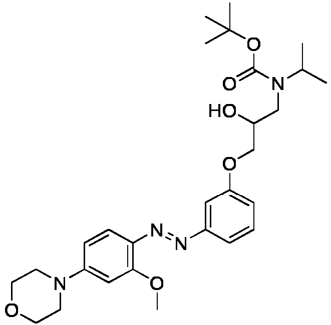

According to method H, from 28 mg of intermediate **(6m)** (0.08 mmol) and 2-methoxy-4-morpholinoaniline, 9.3 mg of intermediate **(8m-9)** were obtained (21% yield) once it was purified by reverse phase chromatography.

¹H-NMR (400 MHz, CDCl₃) δ 7.77 (d, *J* = 9.0 Hz, 1H), 7.51 (d, *J* = 8.0 Hz, 1H), 7.42 (t, *J =* 2.0 Hz, 1H), 7.39 (t, *J* = 8.0 Hz, 1H), 6.98 (ddd, *J* = 8.0, 2.5, 1.0 Hz, 1H), 6.55 (dd, *J* = 9.0, 2.5 Hz, 1H), 6.51 (d, *J* = 2.5 Hz, 1H), 4.12 (br, 2H), 4.05 (s, 3H), 4.04-3.92 (m, 1H), 3.93-3.88 (m, 4H), 3.44 (br, 2H), 3.39-3.33 (m, 4H), 3.24-3.10 (m, 1H), 1.51 (s, 9H), 1.21 (d, *J* = 6.9 Hz, 3H), 1.17 (d, *J* = 6.7 Hz, 3H).

HPLC-MS: retention time= 4.45 min; m/z: 529 [M+1]⁺.

### tert-Butyl(3-(3-((2-(dimethylamino)phenyl)diazenyl)phenoxy)-2-hydroxypropyl) (isopropyl)carbamate (8m-10)

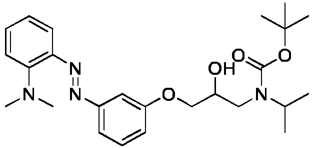

According to method H, from 28 mg of intermediate **(6m)** (0.08 mmol) and *N1,N1-*dimethylbenzene-1,2-diamine, 8.5 mg of intermediate **(8m-10**) were obtained (22% yield) once it was purified by reverse phase chromatography.

HPLC-MS: retention time= 4.49 min; m/z: 457 [M+1]⁺.

### tert-Butyl(2-hydroxy-3-(3-((2-(piperidin-1-yl)phenyl)diazenyl)phenoxy)propyl) (isopropyl)carbamate (8m-11)

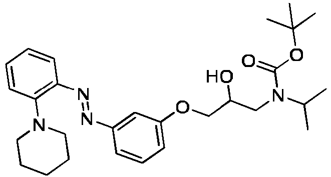

According to method H, from 28 mg of intermediate (**6m**) (0.08 mmol) and 2-(piperidin-1-yl)aniline, 28.6 mg of intermediate (**8m-11**) were obtained (70% yield) once it was purified by reverse phase chromatography.

HPLC-MS: retention time= 5.16 min; m/z: 497 [M+1]⁺.

### tert-Butyl(2-hydroxy-3-(3-((2-morpholinophenyl)diazenyl)phenoxy)propyl) (isopropyl)carbamate (8m-12)

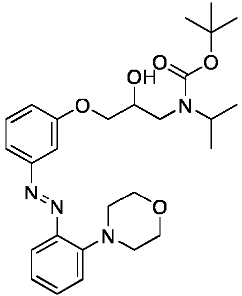

According to method H, from 28 mg of intermediate **(6m)** (0.08 mmol) and 2-morpholinoaniline, 30.2 mg of intermediate **(8m-12)** were obtained (73% yield) once it was purified by reverse phase chromatography.

HPLC-MS: retention time= 4.87 min; m/z: 499 [M+1]⁺.

### tert-Butyl(2-hydroxy-3-(3-((3-morpholinophenyl)diazenyl)phenoxy)propyl) (isopropyl)carbamate (8m-19)

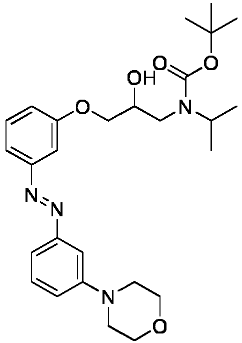

According to method H, from 28 mg of intermediate **(6m)** (0.08 mmol) and 2-morpholinoaniline, 6.0 mg of intermediate **(8m-19)** were obtained (15% yield) once it was purified by reverse phase chromatography.

HPLC-MS: retention time= 5.21 min; m/z: 499 [M+1]⁺.

### tert-Butyl(2-hydroxy-3-(3-((4-(amino)phenyl)diazenyl)phenoxy)propyl)(isopropy I)carbamate (8m-13)

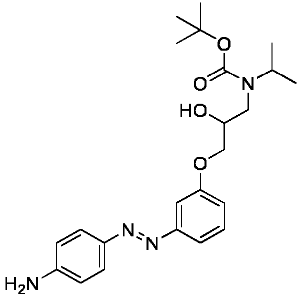

According to method H, from 28 mg of intermediate (**6m**) (0.08 mmol) and benzene-1,4-diamine, 4.3 mg of intermediate (**8m-13)** were obtained (12% yield) once it was purified by reverse phase chromatography.

HPLC-MS: retention time= 4.25 min; m/z: 429 [M+1]⁺.

### tert-Butyl(3-(3-((4-(ethyl(2-hydroxyethyl)amino)phenyl)diazenyl)phenoxy)-2-hydroxypropyl)(isopropyl)carbamate (8m-14)

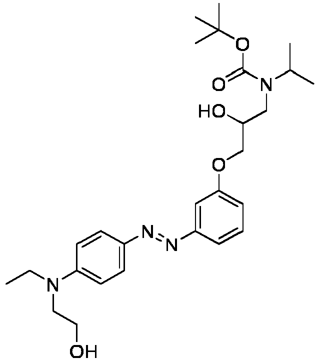

According to method H, from 28 mg of intermediate (**6m**) (0.08 mmol) and 2-((4-aminophenyl)(ethyl)amino)ethan-1-ol, 4.1 mg of intermediate (**8m-14**) were obtained (10% yield) once it was purified by reverse phase chromatography.

HPLC-MS: retention time= 4.43 min; m/z: 501 [M+1]⁺.

### tert-Butyl(3-(3-((4-(diethylamino)phenyl)diazenyl)phenoxy)-2-hydroxypropyl) (isopropyl)carbamate (8m-15)

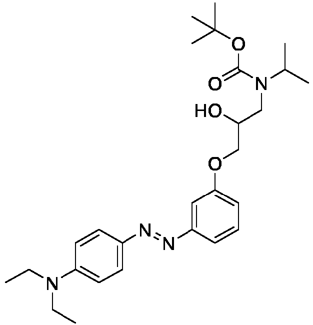

According to method H, from 28 mg of intermediate (**6m**) (0.08 mmol) and *N1,N1-*diethylbenzene-1,4-diamine, 6.5 mg of intermediate (**8m-15**) were obtained (16% yield) once it was purified by reverse phase chromatography.

HPLC-MS: retention time= 5.35 min; m/z: 485 [M+1]⁺.

### tert-Butyl(2-hydroxy-3-(2-((4-(methylamino)phenyl)diazenyl)phenoxy)propyl) (isopropyl)carbamate (8o-2)

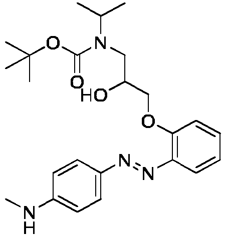

According to method H, from 28 mg of intermediate **(6o)** (0.08 mmol) and *N-*methylaniline, 1.8 mg of intermediate **(8o-2)** were obtained (5% yield) once it was purified by reverse phase chromatography.

HPLC-MS: retention time= 4.07 min; m/z: 443 [M+1]⁺.

### tert-Butyl(3-(2-((4-(azetidin-1-yl)phenyl)diazenyl)phenoxy)-2-hydroxypropyl) (isopropyl)carbamate (8o-3)

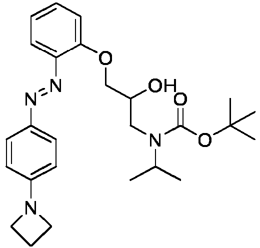

According to method H, from 38.0 mg of intermediate **(6o)** (0.07 mmol) and 4-(azetidin-1-yl)aniline, 20.1 mg of intermediate **(8o-3)** were obtained (38% yield) once it was purified by reverse phase chromatography.

HPLC-MS: retention time= 5.05 min; m/z: 469 [M+1]⁺.

### tert-Butyl(2-hydroxy-3-(2-((4-(pyrrolidin-1-yl)phenyl)diazenyl)phenoxy)propyl) (isopropyl)carbamate (8o-4)

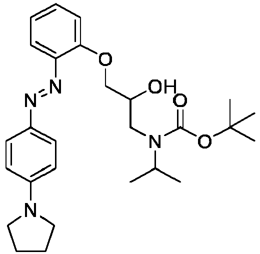

According to method H, from 22.5 mg of intermediate **(6o)** (0.07 mmol) and 4-(pyrrolidin-1-yl)aniline, 10 mg of intermediate **(8o-4)** were obtained (26% yield) once it was purified by reverse phase chromatography.

HPLC-MS: retention time= 5.42 min; m/z: 483 [M+1]⁺.

### tert-Butyl(2-hydroxy-3-(2-((4-(piperidin-1-yl)phenyl)diazenyl)phenoxy)propyl) (isopropyl)carbamate (8o-5)

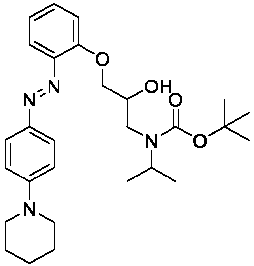

According to method H, from 24.5 mg of intermediate **(6o)** (0.14 mmol) and 4-(piperidin-1-yl)aniline, 24.1 mg of intermediate **(8o-5)** were obtained (35% yield) once it was purified by reverse phase chromatography.

¹H-NMR (400 MHz, CDCl₃) δ 7.86 (d, *J* = 9.1 Hz, 2H), 7.63 (d, *J* = 7.4 Hz, 1H), 7.37 (ddd, *J* = 8.4, 7.4, 1.7 Hz, 1H), 7.12 (dd, *J* = 8.4, 1.2 Hz, 1H), 7.01-7.02 (br, 1H), 6.96 (d, *J* = 9.1 Hz, 2H), 4.35-3.99 (m, 5H), 3.60-3.50 (br,1H), 3.38 (t, *J* = 5.3 Hz, 5H), 1.76-1.64 (m, 6H), 1.50 (s, 9H), 1.18 (d, *J* = 6.8 Hz, 3H), 1.13 (br, 3H).

HPLC-MS: retention time= 5.45 min; m/z: 497 [M+1]⁺.

### tert-Butyl(2-hydroxy-3-(2-((4-morpholinophenyl)diazenyl)phenoxy)propyl) (isopropyl)carbamate (8o-6)

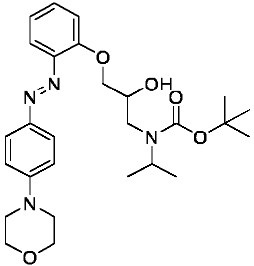

According to method H, from 22.6 mg of intermediate **(6o)** (0.13 mmol), and 4-morpholinoaniline, 39.1 mg of intermediate **(8o-6)** were obtained (61% yield) once it was purified by reverse phase chromatography.

HPLC-MS: retention time= 5.70 min; m/z: 499 [M+1]⁺.

### tert-Butyl(2-hydroxy-3-(2-((4-(4-methylpiperazin-1-yl)phenyl)diazenyl)phenoxy) propyl)(isopropyl)carbamate (8o-7)

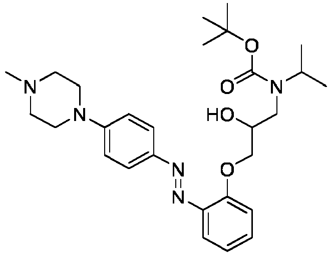

According to method H, from 28 mg of intermediate **(6o)** (0.08 mmol) and 4-(4-methylpiperazin-1-yl)aniline, 38.4 mg of intermediate **(8o-7),** as formate salt, were obtained (84% yield) once it was purified by reverse phase chromatography.

¹H-NMR (400 MHz, CDCl₃) **δ δ 8.33** (br, 1H), 7.88 (d, *J* = 9.0 Hz, 2H), 7.64 (d, *J* = 8.0 Hz, 1H), 7.40 (ddd, *J* = 8.7, 7.3, 1.7 Hz, 1H), 7.13 (dd, *J* = 8.3, 1.2 Hz, 1H), 7.07 (t, *J* = 7.6 Hz, 1H), 6.98 (d, *J* = 9.0 Hz, 2H), 4.33-3.99 (m, 4H), 3.63-3.47 (m, 5H), 3.43 (br, 1H), 2.98 (t, *J* = 5.0 Hz, 4H), 2.60 (s, 3H), 1.50 (s, 9H), 1.17 (d, J = 6.8 Hz, 3H), 1.12 (d, *J* = 6.4 Hz, 3H).

HPLC-MS: retention time= 2.59 min; m/z: 512 [M+1]⁺.

### tert-Butyl(2-hydroxy-3-(2-((2-methoxy-4-morpholinophenyl)diazenyl)phenoxy) propyl)(isopropyl)carbamate (8o-9)

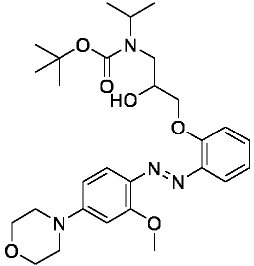

According to method H, from 28 mg of intermediate **(6o)** (0.08 mmol) and 2-methoxy-4-morpholinoaniline, 4.8 mg of intermediate **(8o-9)** were obtained (12% yield) once it was purified by reverse phase chromatography.

HPLC-MS: retention time= 3.84 min; m/z: 529 [M+1]⁺.

### tert-Butyl(3-(2-((2-(dimethylamino)phenyl)diazenyl)phenoxy)-2-hydroxypropyl) (isopropyl)carbamate (8o-10)

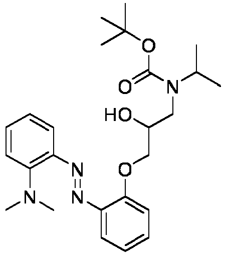

According to method H, from 28 mg of intermediate **(6o)** (0.08 mmol) and *N1,N1-*dimethylbenzene-1,2-diamine, 28 mg of intermediate **(8o-10)** were obtained (74% yield) once it was purified by reverse phase chromatography.

HPLC-MS: retention time= 3.55 min; m/z: 457 [M+1]⁺.

### tert-Butyl(2-hydroxy-3-(2-((2-(piperidin-1-yl)phenyl)diazenyl)phenoxy)propyl) (isopropyl)carbamate (8o-11)

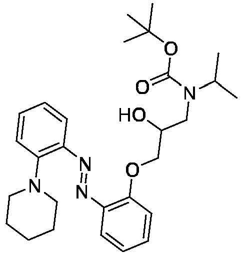

According to method H, from 28 mg of intermediate **(6o)** (0.08 mmol) and 2-(piperidin-1-yl)aniline, 15.2 mg of intermediate **(8o-11)** were obtained (37% yield) once it was purified by reverse phase chromatography.

HPLC-MS: retention time= 4.32 min; m/z: 497 [M+1]⁺.

### tert-Butyl(2-hydroxy-2-(3-((2-morpholinophenyl)diazenyl)phenoxy)propyl) (isopropyl)carbamate (8o-12)

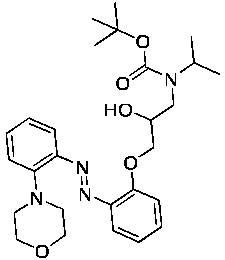

According to method H, from 28 mg of intermediate **(6o)** (0.08 mmol) and 2-morpholinoaniline, 15.0 mg of intermediate **(8o-12)** were obtained (36% yield) once it was purified by reverse phase chromatography.

HPLC-MS: retention time= 4.189 min; m/z: 499 [M+1]⁺.

### tert-Butyl(2-hydroxy-3-(2-((4-(amino)phenyl)diazenyl)phenoxy)propyl)(isopropy l)carbamate (8o-13)

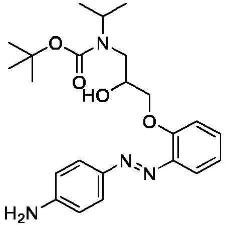

According to method H, from 28 mg of intermediate **(6o)** (0.08 mmol) and benzene-1,4-diamine, 4.6 mg of intermediate **(8o-13)** were obtained (13% yield) once it was purified by reverse phase chromatography.

HPLC-MS: retention time= 4.25 min; m/z: 429 [M+1]⁺.

### tert-Butyl(3-(2-((4-(ethyl(2-hydroxyethyl)amino)phenyl)diazenyl)phenoxy)-2-hydroxypropyl)(isopropyl)carbamate (8o-14)

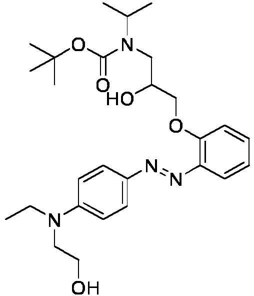

According to method H, from 28 mg of intermediate **(6o)** (0.08 mmol) and 2-((4-aminophenyl)(ethyl)amino)ethan-1-ol, 15.1 mg of intermediate **(8o-14)** were obtained (36% yield) once it was purified by reverse phase chromatography.

¹H-NMR (400 MHz, CDCl₃) δ 7.85 (d, *J =* 9.1 Hz, 2H), 7.63 (d, *J =* 8.0 Hz, 1H), 7.36 (ddd, *J =* 8.4, 8.0, 1.7 Hz, 1H), 7.11 (dd, *J =* 8.4, 1.1 Hz, 1H), 7.07 (br, 1H), 6.79 (d, J = 9.1 Hz, 2H), 4.28-4.09 (m, 4H), 3.87 (t, *J* = 6.0 Hz, 2H), 3.59 (t, *J* = 6.0 Hz, 2H), 3.54 (q, *J* = 7.0 Hz, 2H), 3.58-3.52 (m, 1H), 3.42 (br, 1H), 1.50 (s, 9H), 1.25 (t, *J =* 7.0 Hz, 3H), 1.18 (d, *J* = 6.8 Hz, 3H), 1.14 (d, *J =* 5.3 Hz, 3H).

HPLC-MS: retention time= 3.87 min; m/z: 501 [M+1]⁺.

### tert-Butyl(3-(2-((4-(diethylamino)phenyl)diazenyl)phenoxy)-2-hydroxypropyl) (isopropyl)carbamate (8o-15)

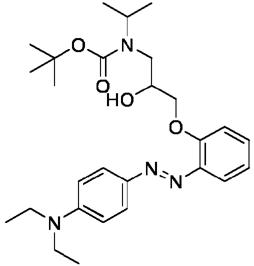

According to method H, from 28 mg of intermediate **(6o)** (0.08 mmol) and *N1,N1-*diethylbenzene-1,4-diamine, 19.6 mg of intermediate **(8o-15)** were obtained (49% yield) once it was purified by reverse phase chromatography.

¹H-NMR (400 MHz, CDCl₃) δ 7.86 (d, *J =* 9.2 Hz, 2H), 7.63 (d, *J =* 7.9 Hz, 1H), 7.34 (ddd, *J =* 8.4, 7.3, 1.8 Hz, 1H), 7.11 (dd, *J* = 8.4, 1.2 Hz, 1H), 7.11-7.02 m, 1H), 6.72 (d, *J* = 9.2 Hz, 2H), 4.30-4.00 (m, 4H), 3.55-3.22 (m, 3H), 3.47 (q, *J* = 7.1 Hz, 4H), 1.49 (s, 9H), 1.25 (t, *J =* 7.1 Hz, 6H), 1.18 (d, *J =* 6.9 Hz, 3H), 1.14 (br, 3H).

HPLC-MS: retention time= 4.89 min; m/z: 485 [M+1]⁺.

### tert-Butyl(2-hydroxy-3-(2-((3-morpholinophenyl)diazenyl)phenoxy)propyl) (isopropyl)carbamate (8o-19)

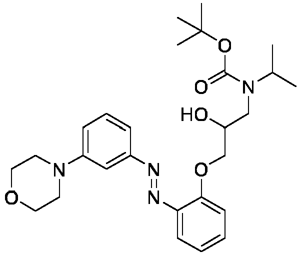

According to method H, from 28 mg of intermediate **(6o)** (0.08 mmol) and 3-morpholinoaniline, 0.5 mg of intermediate **(8o-19)** were obtained (1% yield) once it was purified by reverse phase chromatography.

HPLC-MS: retention time= 4.29 min; m/z: 499 [M+1]⁺.

### Example I-1

### (S)-1-(4-((4-(Dimethylamino)phenyl)diazenyl)phenoxy)-3-(isopropylamino)prop an-2-ol.

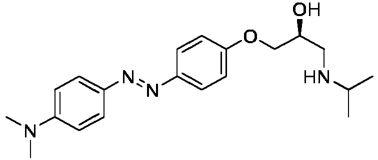

According to method C, from 25 mg of intermediate **((*S*)-11p)** (0.075 mmol), 951 mg of compound **(I-1)** were obtained (99% yield) as an orange solid once it was purified by column chromatography (95:5 EtOAc: MeOH).

¹H-NMR (400 MHz, DMSO-d₆) δ 7.80-7.70 (m, 4H), 7.07 (d, *J* = 9.1 Hz, 2H), 6.82 (d, *J* = 9.1 Hz, 2H), 4.05 (dd, J = 9.8, 4.5 Hz, 1H), 3.95 (dd, *J* = 9.8, 6.8 Hz, 1H), 3.91-3.84 (m, 1H), 3.04 (s, 1H), 2.79-2.66 (m, 2H), 2.59 (dd, *J* = 11.7, 6.8 Hz, 1H), 0.99 (dd, *J* = 6.2, 1.5 Hz, 6H).

[α]^{25D}= + 14.6 (c = 1.0, MeOH).

### Example I-2

### (R)-1-(4-((4-(Dimethylamino)phenyl)diazenyl)phenoxy)-3-(isopropylamino) propan-2-ol.

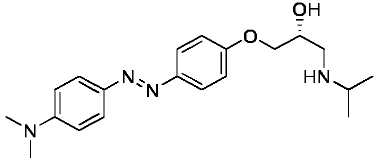

According to method C, from 201 mg of intermediate **((*R*)-11p)** (0.075 mmol), 44 mg of compound **(I-2)** were obtained (18% yield) as an orange solid once it was purified by column chromatography (95:5 EtOAc:MeOH).

Chiral HPLC: t_{R} = 14.5 min, ee 99 %.

### Example I-3

### 1-(Isopropylamino)-3-(4-((4-(methylamino)phenyl)diazenyl)phenoxy)propan-2-ol.

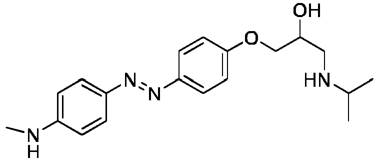

According to method L, from 2.6 mg of intermediate **(8p-2)** (0.01 mmol), 2.80 mg of compound **(I-3)** were obtained (99% yield) as trifluoroacetate salt.

¹H-NMR (400 MHz, CDCl₃) δ 7.77 (d, *J* = 6.0 Hz, 2H), 7.74 (d, *J* = 6.0 Hz, 2H), 6.95 (d, *J =* 9.0 Hz, 2H), 6.63 (d, *J* = 9.0 Hz, 2H), 4.36-4.28 (m, 1H), 4.08 (dd, *J* = 9.6, 4.4 Hz, 1H), 3.98 (dd, *J* = 9.6, 5.9 Hz, 1H), 3.37 (h, *J* = 6.6 Hz, 1H), 3.20 (dd, *J* = 12.3, 2.6 Hz, 1H), 3.03 (dd, *J* = 12.3, 11.5 Hz, 1H), 2.87 (s, 3H), 1.36 (d, *J* = 6.6 Hz, 3H), 1.35 (d, *J* = 6.6 Hz, 3H).

HPLC-MS: retention time= 2.82 min; m/z: 343 [M+1]⁺.

### Example I-4

### 1-(4-((4-(Azetidin-1-yl)phenyl)diazenyl)phenoxy)-3-(isopropylamino) propan-2-ol.

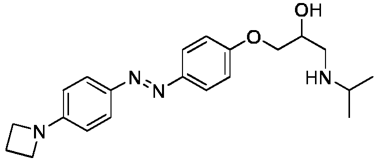

According to method L, from 4.8 mg of intermediate **(8p-3)** (0.01 mmol), 2.32 mg of compound **(I-4)** were obtained (38% yield) as formate salt once it was purified by reverse phase.

¹H-NMR (400 MHz, CD₃OD) δ 8.56 (br, 1H), 7.81 (d, *J* = 8.8 Hz, 2H), 7.78 (d, *J* = 8.8 Hz, 2H), 7.10 (d, *J =* 8.8 Hz, 2H), 6.52 (d, *J =* 8.8 Hz, 2H), 4.31-4.23 (m, 1H), 4.15 (dd, *J* = 9.8,, 5.6 Hz, 1H), 4.11 (dd, *J* = 9.8, 5.6 Hz, 1H), 4.03 (t, *J* = 7.3 Hz, 4H), 3.44 (h, *J* = 6.6 Hz, 1H), 3.29 (dd, *J* = 12.6, 3.2 Hz, 1H), 3.16 (dd, *J* = 12.6, 9.6 Hz, 1H), 2.46 (p, *J* = 7.3 Hz, 2H), 1.38 (d, J = 6.5 Hz, 3H), 1.37 (d, J = 6.5 Hz, 3H).

HPLC-MS: retention time= 3.17 min; m/z: 369 [M+1]⁺.

### Example I-5

### 1-(Isopropylamino)-3-(4-((4-(pyrrolidin-1-yl)phenyl)diazenyl)phenoxy)propan-2-ol.

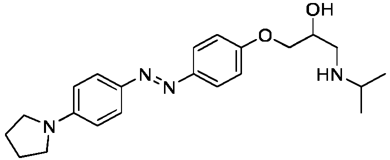

According to method L, from 4.1 mg of intermediate **(8p-4)** (0.01 mmol), 1.07 mg of compound **(I-5)** were obtained (29% yield) as formate salt once it was purified by reverse phase.

HPLC-MS: retention time= 3.17 min; m/z: 383 [M+1]⁺.

### Example I-6

### 1-(Isopropylamino)-3-(4-((4-(piperidin-1-yl)phenyl)diazenyl)phenoxy)propan-2-ol.

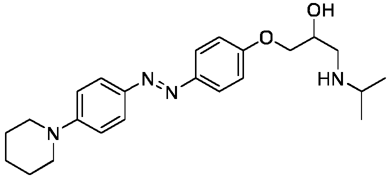

According to method L, from 2.1 mg of intermediate **(8p-5)** (0.01 mmol), 2.2 mg of compound **(I-6)** were obtained (99% yield) as trifluoroacetate salt.

HPLC-MS: retention time= 3.09 min; m/z: 397 [M+1]⁺.

### Example I-7

### 1-(Isopropylamino)-3-(4-((4-morpholinophenyl)diazenyl)phenoxy)propan-2-ol.

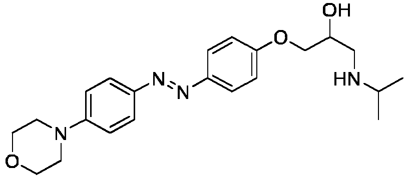

According to method L, from 4.6 mg of intermediate **(8p-6)** (0.01 mmol), 4.85 mg of compound **(I-7)** were obtained (99% yield) as trifluoroacetate salt.

HPLC-MS: retention time= 2.88 min; m/z: 399 [M+1]⁺.

### Example I-8

### 1-(isopropylamino)-3-(4-((4-(4-methylpiperazin-1-yl)phenyl)diazenyl)phenoxy) propan-2-ol (I-8)

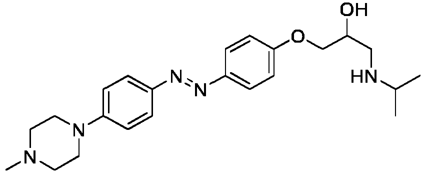

According to method L, from 1.5 mg of intermediate **(8p-7)** (0.01 mmol), 0.71 mg of compound **(I-8)** were obtained (43% yield) as trifluoroacetate salt.

HPLC-MS: retention time= 2.39 min; m/z: 412 [M+1]⁺.

### Example I-9

### 1-(4-((4-(Dimethylamino)-2-methoxyphenyl)diazenyl)phenoxy)-3-(isopropylami no)propan-2-ol.

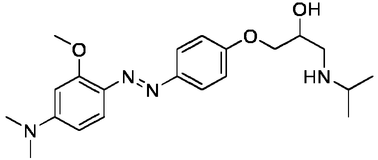

According to method L, from 6.6 mg of intermediate **(8p-8)** (0.01 mmol), 6.37 mg of compound **(I-9)** were obtained (94% yield) as trifluoroacetate salt.

HPLC-MS: retention time= 2.64 min; m/z: 387 [M+1]⁺.

### Example I-10

### 1-(Isopropylamino)-3-(4-((2-methoxy-4-morpholinophenyl)diazenyl)phenoxy)pr opan-2-ol.

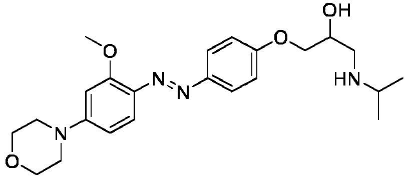

According to method L, from 5.7 mg of intermediate **(8p-9)** (0.01 mmol), 5.46 mg of compound **(I-10)** were obtained (93% yield) as trifluoroacetate salt.

HPLC-MS: retention time= 2.78 min; m/z: 429 [M+1]⁺.

### Example I-11

### 1-(4-((2-(Dimethylamino)phenyl)diazenyl)phenoxy)-3-(isopropylamino)propan-2-ol

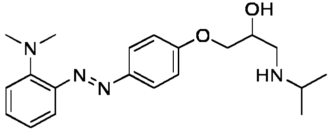

According to method L, from 1.7 mg of intermediate **(8p-10)** (0.01 mmol), 2.03 mg of compound **(I-11)** were obtained (99% yield) as trifluoroacetate salt.

HPLC-MS: retention time= 2.51 min; m/z: 357 [M+1]⁺.

### Example I-12

### 1-(Isopropylamino)-3-(4-((2-(piperidin-1-yl)phenyl)diazenyl)phenoxy)propan-2-ol.

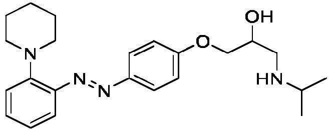

According to method L, from 7.2 mg of intermediate **(8p-11)** (0.01 mmol), 7.32 mg of compound **(I-12)** were obtained (99% yield) as trifluoroacetate salt.

HPLC-MS: retention time= 2.71 min; m/z: 397 [M+1]⁺.

### Example I-13

### 1-(Isopropylamino)-3-(4-((2-morpholinophenyl)diazenyl)phenoxy)propan-2-ol

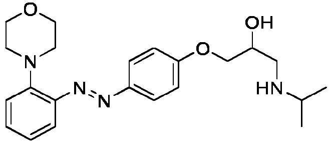

According to method L, from 5.9 mg of intermediate **(8p-12)** (0.01 mmol), 6.1 mg of compound **(I-13)** were obtained (99% yield) as trifluoroacetate salt.

HPLC-MS: retention time= 2.87 min; m/z: 399 [M+1]⁺.

### Example I-14

### 1-(4-((4-Aminophenyl)diazenyl)phenoxy)-3-(isopropylamino)propan-2-ol.

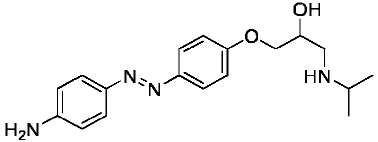

According to method L, from 4 mg of intermediate (8p-13) (0.01 mmol), 4.1 mg of compound **(I-14)** were obtained (87% yield) as trifluoroacetate salt.

HPLC-MS: retention time= 2.62 min; m/z: 329 [M+1]⁺.

### Example I-15

### 1-(4-((4-(Ethyl(2-hydroxyethyl)amino)phenyl)diazenyl)phenoxy)-3-(isopropylamino)propan-2-ol.

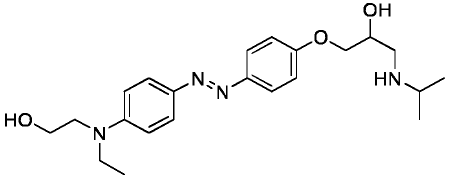

According to method L, from 4.2 mg of intermediate **(8p-14)** (0.01 mmol), 4.0 mg of compound **(I-15)** were obtained (93% yield) as trifluoroacetate salt.

1H-NMR (400 MHz, CDCl₃) **δ 7.78** (d, *J* = 8.8 Hz, 4H), 6.96 (d, *J* = 9.0 Hz, 2H), 6.79 (d, *J* = 9.0 Hz, 2H), 4.37-4.29 (m, 1H), 4.10 (dd, *J* = 9.6, 4.8 Hz, 2H), 3.99 (dd, *J* = 9.6, 6.1 Hz, 1H), ), 3.76 (t, *J =* 6.2 Hz, 2H), 3.54 (t, *J =* 6.2 Hz, 2H), 3.52-3.48 (m, 1H), 3.49 (q, *J* = 7.1 Hz, 2H), 3.38 (dd, *J* = 12.4, 7.2 Hz,1H), 3.21 (dd, *J* = 12.4, 2.9 Hz, 1H), 1.36 (d, *J* = 6.6 Hz, 3H), 1.34 (d, *J =* 6.6 Hz, 3H), 1.21 (d, *J* = 7.1 Hz, 6H).

HPLC-MS: retention time= 2.78 min; m/z: 401 [M+1]⁺.

### Example I-16

### 1-(4-((4-(Diethylamino)phenyl)diazenyl)phenoxy)-3-(isopropylamino)propan-2-ol (I-16)

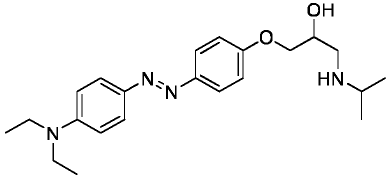

According to method L, from 3.9 mg of intermediate **(8p-15)** (0.01 mmol), 1.93 mg of compound **(I-16)** were obtained (56% yield) as formate salt once it was purified by reverse phase.

1H-NMR (400 MHz, CD₃OD) **δ 8.55** (br, 1H), 7.80 (d, *J =* 8.9 Hz, 2H), 7.77 (d, *J =* 8.9 Hz, 2H), 7.10 (d, *J* = 9.2 Hz, 2H), 6.80 (d, *J* = 9.2 Hz, 2H), 4.32-4.23 (m, 1H), 4.15 (dd, *J* = 9.8, 5.1 Hz, 1H), 4.10 (dd, *J* = 9.8, 5.4 Hz, 1H), 3.52 (q, *J* = 7.1 Hz, 4H), 3.45 (h, *J* = 6.6 Hz, 1H), 3.29 (dd, *J* = 12.6, 3.0 Hz, 1H), 3.17 (dd, *J* = 12.6, 9.5 Hz, 1H), 1.38 (d, *J* = 6.6 Hz, 3H), 1.37 (d, *J =* 6.6 Hz, 3H), 1.24 (t, *J =* 7.1 Hz, 6H).

HPLC-MS: retention time= 3.05 min; m/z: 395 [M+1]⁺.

### Example I-17

### (S)-1-(3-((4-(Dimethylamino)phenyl)diazenyl)phenoxy)-3-(isopropylamino)prop an-2-ol.

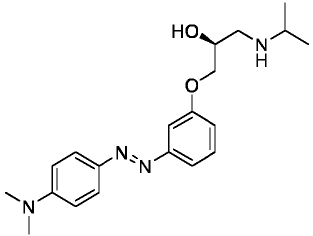

According to method C, from 130 mg of intermediate **((*S*)-11m)** (0.48 mmol), 137 mg of compound **(I-17)** were obtained (88% yield) as an orange solid once it was purified by column chromatography (95:5 EtOAc:MeOH).

1H-NMR (400 MHz, CDCl₃) δ 7.87 (d, *J* = 9.1 Hz, 2H), 7.46 (dt, *J* = 8.0, 1.5 Hz, 1H), 7.38 (t, *J =* 1.5 Hz, 1H), 7.35 (t, *J =* 8.0 Hz, 1H), 6.96 (dd, *J* = 8.0, 1.5, 1H), 6.76 (d, *J* = 9.1 Hz, 2H), 4.17-4.02 (m, 3H), 3.09 (s, 6H), 2.98-2.76 (m, 4H), 1.14 (d, *J =* 6.3 Hz, 6H).

[α]^{25D}= - 6.2 (c = 1.0, MeOH).

Chiral HPLC: t_{R} = 19.9 min, ee 91 %.

### Example I-18

### 1-(Isopropylamino)-3-(3-((4-(methylamino)phenyl)diazenyl)phenoxy)propan-2-ol.

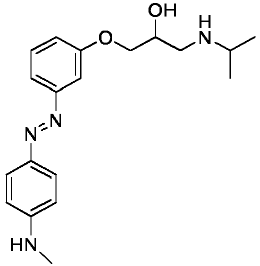

According to method L, from 3.3 mg of intermediate **(8m-2)** (0.01 mmol), 3.88 mg of compound **(I-18)** were obtained (99% yield) as trifluoroacetate salt.

¹H-NMR (400 MHz, CDCl₃) δ 7.81 (d, *J =* 8.8 Hz, 2H), 7.46 (d, *J =* 8.0 Hz, 1H), 7.38 (t, *J =* 8.0 Hz, 1H), 7.34 (d, *J* = 2.0 Hz, 1H), 6.93 (dd, *J* = 8.0, 2.0 Hz, 1H), 6.66 (d, *J* = 8.8 Hz, 2H), 4.42-4.31 (m, 1H), 4.13 (dd, *J =* 9.7, 4.5 Hz, 1H), 4.02 (dd, *J* = 9.7, 6.1 Hz, 1H), 3.44-3.34 (m, 1H), 3.23 (dd, *J* = 12.2, 2.8 Hz, 1H), 3.07 (dd, *J* = 12.2 10.0 Hz, 1H), 2.91 (s, 3H), 1.38 (d, *J* = 6.6, Hz, 3H), 1.36 (d, *J* = 6.6, Hz, 3H)..

HPLC-MS: retention time= 2.87 min; m/z: 172 [M+2]²⁺.

### Example I-19

### 1-(3-((4-(Azetidin-1-yl)phenyl)diazenyl)phenoxy)-3-(isopropylamino)propan-2-ol.

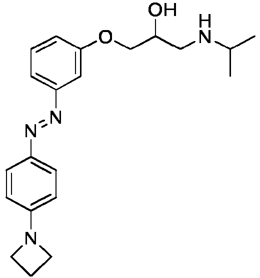

According to method L, from 10 mg of intermediate **(8m-3)** (0.07 mmol), 2.07 mg of compound **(I-19)** were obtained (26% yield) once it was purified by reverse phase chromatography.

HPLC-MS: retention time= 3.10 min; m/z: 369 [M+1]⁺.

### Example I-20

### 1-(Isopropylamino)-3-(3-((4-(pyrrolidin-1-yl)phenyl)diazenyl)phenoxy)propan-2-ol.

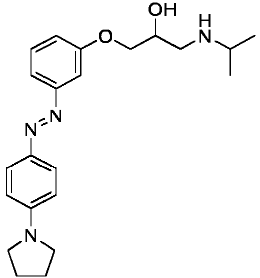

According to method L, from 10 mg of intermediate **(8m-4)** (0.07 mmol), 3.92 mg of compound **(I-20)** were obtained (50% yield) as formate salt once it was purified by reverse phase chromatography

HPLC-MS: retention time= 3.21 min; m/z: 383 [M+1]⁺.

### Example I-21

### 1-(Isopropylamino)-3-(3-((4-(piperidin-1-yl)phenyl)diazenyl)phenoxy)propan-2-ol.

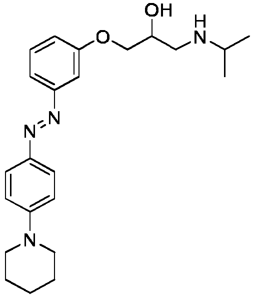

According to method L, from 10 mg of intermediate **(8m-5)** (0.07 mmol), 2.17 mg of compound **(I-21)** were obtained (27% yield) as formate once it was purified by reverse phase chromatography

HPLC-MS: retention time= 3.22 min; m/z: 397 [M+1]⁺.

### Example I-22

### 1-(Isopropylamino)-3-(3-((4-morpholinophenyl)diazenyl)phenoxy)propan-2-ol.

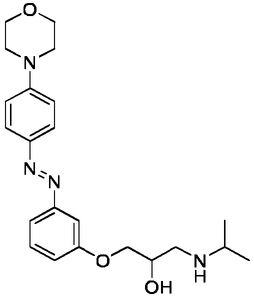

According to method L, from 10 mg of intermediate (8m-6) (0.07 mmol), 1.17 mg of compound (I-22) were obtained (11% yield) as formate salt once it was purified by reverse phase chromatography

HPLC-MS: retention time= 2.97 min; m/z: 399 [M+1]⁺.

### Example I-23

### 1-(Isopropylamino)-3-(3-((4-(4-methylpiperazin-1-yl)phenyl)diazenyl)phenoxy) propan-2-ol.

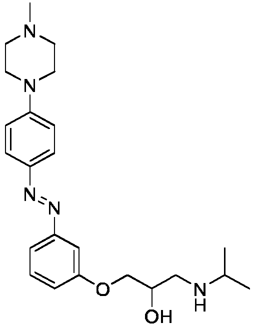

According to method L, from 12 mg of intermediate **(8m-7)** (0.02 mmol), 12.0 mg of compound **(I-23)** were obtained (80% yield) as trifluoroacetate salt.

¹H-NMR (400 MHz, CDCl₃) δ 7.85 (d, *J =* 9.0 Hz, 2H), 7.49 (d, *J =* 7.8 Hz, 1H), 7.37 (t, *J =* 7.8 Hz, 1H), 7.35 (d, *J* = 2.1 Hz, 1H), 6.99-6.96 (m, 1H), 6.98 (d, *J* = 9.0 Hz, 2H), 4.36-4.29 (m, 1H), 4.12 (dd, *J* = 9.6, 4.5 Hz, 1H), 4.01 (dd, *J* = 9.6, 6.1 Hz, 1H), 3.56 (d, *J* = 4.9 Hz, 1H), 3.42-3.28 (m, 5H), 3.10-3.02 (m, 2H), 2.87 (s, 3H), 1.35 (d, *J* = 6.6 Hz, 3H), 1.34 (d, *J* = 6.6 Hz, 3H).

HPLC-MS: retention time= 2.43 min; m/z: 412 [M+1]⁺.

### Example I-24

### 1-(isopropylamino)-3-(3-((2-methoxy-4-morpholinophenyl)diazenyl)phenoxy) propan-2-ol.

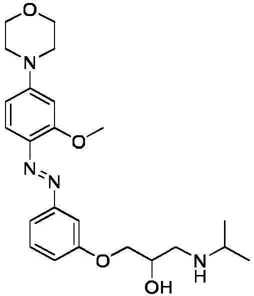

According to method L, from 9.3 mg of intermediate **(8m-9)** (0.02 mmol), 9.5 mg of compound **(I-24)** were obtained (99% yield) as trifluoroacetate salt.

¹H-NMR (400 MHz, CDCl₃) δ 7.67 (d, *J* = 9.2 Hz, 1H), 7.38 (d, *J* = 8.0 Hz, 1H), 7.28 (t, *J* = 8.0 Hz, 1H), 7.26 (s, 1H), 6.85 (dd, *J* = 8.0, 2.5 Hz, 1H), 6.53 (dd, *J* = 9.2, 2.5 Hz, 1H), 6.39 (d, *J* = 2.5 Hz, 1H), 4.28-4.23 (m, 1H), 4.05 (dd, *J* = 9.7, 4.5 Hz, 1H), 3.97-3.94 (m, 1H), 3.96 (s, 3H), 3.80 (t, *J* = 5.0 Hz, 4H), 3.37 (t, *J* = 5.0 Hz, 4H), 3.34-3.27 (m, 2H), 3.16 (dd, *J* = 12.5, 3.0 Hz, 1H), 3.00 (dd, *J* = 12.5, 9.9 Hz, 1H), 1.30 (d, *J* = 6.4 Hz, 3H), 1.28 (d, *J* = 6.4 Hz, 3H).

HPLC-MS: retention time= 2.82 min; m/z: 429 [M+1]⁺.

### Example I-25

### 1-(3-((2-(Dimethylamino)phenyl)diazenyl)phenoxy)-3-(isopropylamino)propan-2-ol.

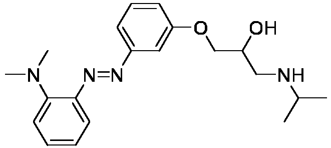

According to method L, from 10.5 mg of intermediate **(8m-10)** (0.02 mmol), 11.07 mg of compound **(I-25)** as trifluoroacetate salt were obtained (99% yield).

HPLC-MS: retention time= 2.66 min; m/z: 357 [M+1]⁺.

### Example I-26

### 1-(Isopropylamino)-3-(3-((2-(piperidin-1-yl)phenyl)diazenyl)phenoxy)propan-2-ol.

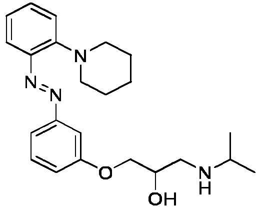

According to method L, from 28.6 mg of intermediate **(8m-11)** (0.02 mmol), 3.52 mg of compound **(I-26)** were obtained (14% yield) as formate salt once it was purified by reverse phase.

1H-NMR (400 MHz, CD₃OD) δ 8.49 (s, 1H), 7.63 (d, *J* = 7.6 Hz, 1H), 7.60 (t, *J* = 8.0 Hz, 1H), 7.56 (t, *J* = 2.4 Hz, 1H), 7.51 (t, *J* = 8.0 Hz, 1H), 7.45 (td, *J* = 7.6, 1.2 Hz, 1H), 7.23 (d, *J* = 8.4 Hz, 1H), 7.16 (dd, *J* = 8.0, 2.4 Hz, 1H), 7.06 (t, *J* = 7.6 Hz, 1H), 4.32-4.27 (m, 1H), 4.18 (dd, *J =* 9.6, 4.9 Hz, 1H), 4.13 (dd, *J =* 9.6, 5.6 Hz, 1H), 3.48 (h, *J* = 6.5 Hz, 1H), 3.38-3.28 (m, 2H), 3.24-3.15 (m, 4H), 1.83 (p, *J* = 5.7 Hz, 2H), 1.67 (p, *J =* 5.7 Hz, 2H), 1.41 (d, *J* = 6.5 Hz, 3H), 1.40 (d, *J* = 6.5 Hz, 3H), 1.39-1.35 (m, 2H).

HPLC-MS: retention time= 2.82 min; m/z: 397 [M+1]⁺.

### Example I-27

### 1-(Isopropylamino)-3-(3-((2-morpholinophenyl)diazenyl)phenoxy)propan-2-ol.

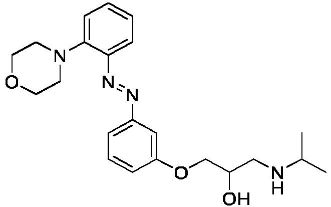

According to method L, from 30.2 mg of intermediate (8m-12) (0.02 mmol), 12.2 mg of compound **(I-27)** were obtained (45% yield) as formate salt, once it was purified by reverse phase chromatography.

HPLC-MS: retention time= 2.90 min; m/z: 399 [(M+1]⁺.

### Example I-28

### 1-(3-((4-Aminophenyl)diazenyl)phenoxy)-3-(isopropylamino)propan-2-ol.

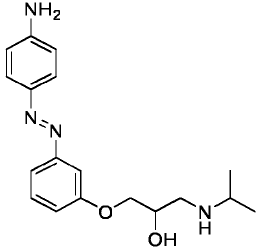

According to method L, from 4.3 mg of intermediate **(8m-13)** (0.02 mmol), 4.52 mg of compound **(I-28)** were obtained (14% yield) as trifluoroacetate salt.

1H-NMR (400 MHz, CDCl₃) δ **7.76** (d, *J* = 8.8 Hz, 2H), 7.46 (dt, *J* = 8.0, 1.2 Hz, 1H), 7.37 (t, *J* = 8.0 Hz, 1H), 7.33 (dd, *J =* 2.1, 1.2 Hz, 1H), 6.94 (ddd, *J* = 8.0, 2.1, 1.2 Hz, 1H), 6.75 (d, *J* = 8.8 Hz, 2H), 4.39-4.31 (m, 1H), 4.12 (dd, *J* = 9.6, 4.5 Hz, 1H), 4.01 (dd, *J* = 9.6, 6.1 Hz, 1H), 3.39 (h, *J* = 6.6 Hz, 1H), 3.22 (dd, *J* = 12.4, 2.9 Hz, 1H), 3.08 (dd, *J* = 12.4, 10.1 Hz, 1H), 1.37 (d, *J* = 6.6 Hz, 3H), 1.36 (d, *J* = 6.6 Hz, 3H)

HPLC-MS: retention time= 2.70 min; m/z: 329 [M+1]⁺.

### Example I-29

### 1-(3-((4-(Ethyl(2-hydroxyethyl)amino)phenyl)diazenyl)phenoxy)-3-(isopropylamino)propan-2-ol.

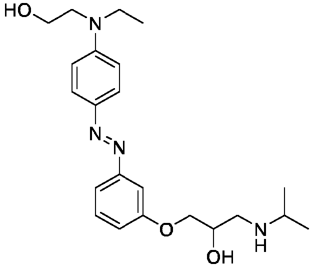

According to method L, from 4.1 mg of intermediate **(8m-14)** (0.01 mmol), 3.06 mg of compound **(I-29)** were obtained (73% yield) as trifluoroacetate salt.

HPLC-MS: retention time= 2.86 min; m/z: 401 [M+1]⁺.

### Example I-30

### 1-(3-((4-(Diethylamino)phenyl)diazenyl)phenoxy)-3-(isopropylamino)propan-2-ol.

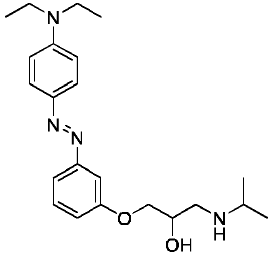

According to method L, from 6.5 mg of intermediate **(8m-15)** (0.01 mmol), 1.21 mg of compound **(I-30)** were obtained (23% yield) as formate salt once it was purified by reverse phase chromatography.

¹H-NMR (400 MHz, CD₃OD) δ 8.55 (br, 1H), 7.82 (d, *J =* 9.2 Hz, 2H), 7.49-7.38 (m, 3H), 7.04 (dt, *J* = 7.5, 2.1 Hz, 1H), 6.82 (d, *J =* 9.2 Hz, 2H), 4.31-4.24 (m, 1H), 4.15 (dd, *J* = 9.8, 5.2 Hz, 1H), 4.11 (dd, *J* = 9.8, 5.6 Hz, 1H), 3.53 (q, *J* = 7.2 Hz, 4H), 3.44 (h, *J =* 6.6 Hz, 2H), 3.30 (dd, *J* = 12.4, 3.1 Hz, 1H), 3.17 (dd, *J* = 12.4, 9.2 Hz, 1H), 1.38 (d, *J* = 6.6 Hz, 3H), 1.36 (d, *J =* 6.6 Hz, 7H), 1.25 (t, *J* = 7.2 Hz, 6H).

HPLC-MS: retention time= 3.14 min; m/z: 385 [M+1]⁺.

### Example I-31

### (S)-1-(2-((4-(Dimethylamino)phenyl)diazenyl)phenoxy)-3-(isopropylamino)prop an-2-ol.

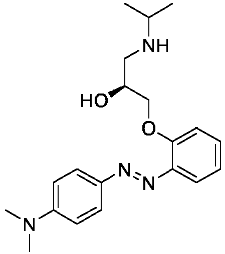

According to method C, from 2.82 g of intermediate **((*S*)-11o)** (4.77 mmol), 360 mg of compound **(I-31)** were obtained (21% yield) as an orange solid once it was purified by reverse phase chromatography.

¹H-NMR (400 MHz, CDCl₃)) δ **7.83** (d, *J =* 9.1 Hz, 2H), 7.62 (dd, *J* = 8.2, 1.7 Hz, 1H), 7.31 (ddd, *J* = 8.2, 7.2, 1.7 Hz, 1H), 7.09 (dd, *J* = 8.2, 1.2 Hz, 1H), 7.05 (ddd, *J* = 8.2, 7.2, 1.2 Hz, 1H), 6.73 (d, *J =* 9.1 Hz, 2H), 4.27-4.21 (m, 1H), 4.19-4.09 (m, 2H), 3.07 (s, 6H), 2.90-2.77 (m, 4H), 1.06 (d, *J* = 6.3 Hz, 6H).

[α]^{25D}= - 278.4 (c = 1.0, MeOH).

Chiral HPLC t_{R} = 12.6 min, ee 99%.

### Example I-32

### 1-(Isopropylamino)-3-(2-((4-(methylamino)phenyl)diazenyl)phenoxy)propan-2-ol.

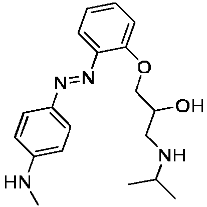

According to method L, from 1.8 mg of intermediate **(8o-2)** (0.04 mmol), 1.37 mg of compound **(I-32)** as trifluoroacetate salt were obtained (74% yield).

HPLC-MS: retention time= 2.90 min; m/z: 343 [M+1]⁺.

### Example I-33

### 1-(2-((4-(Azetidin-1-yl)phenyl)diazenyl)phenoxy)-3-(isopropylamino)propan-2-ol.

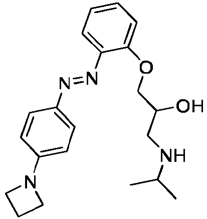

According to method L, from 20.1 mg of intermediate **(8o-3)** (0.04 mmol), 5.86 mg of compound **(I-33)** as formate salt were obtained (37% yield) once it was purified by reverse phase chromatography.

H-NMR (400 MHz, CDCl₃) δ 8.50 (br, 1H), 7.81 (d, *J =* 9.0 Hz, 2H), 7.66 (dd, *J* = 8.2, 1.7 Hz, 1H), 7.36 (td, *J =* 8.2, 7.8, 1.7 Hz, 1H), 7.11-7.03 (m, 2H), 6.95 (d, *J* = 9.0 Hz, 2H), 4.52 (br, 1H), 4.34-4.26 (m, 2H), 3.38-3.36 (m, 5H), 3.32-3.25 (m, 1H), 3.19 (dd, *J=* 12.5, 6.4 Hz, 1H), 1.70-1.67 (m, 2H), 1.25 (d, *J* = 4.6 Hz, 6H).

HPLC-MS: retention time= 3.10 min; m/z: 369 [M+1]⁺.

### Example I-34

### 1-(Isopropylamino)-3-(2-((4-(piperidin-1-yl)phenyl)diazenyl)phenoxy)propan-2-ol.

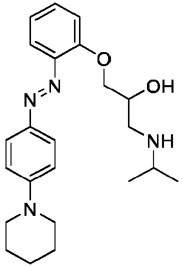

According to method L, from 24.1 mg of intermediate **(8o-5)** (0.05 mmol), 6.94 mg of compound **(I-34)** as formate salt were obtained (36% yield) once it was purified by reverse phase chromatography.

H-NMR (400 MHz, CDCl₃) δ 8.55 (s, 1H), 7.81 (d, *J* = 9.1 Hz, 2H), 7.66 (dd, *J =* 8.4, 2.0 Hz, 1H), 7.36 (td, *J =* 8.0, 1.7 Hz, 1H), 7.07 (td, *J* = 8.0, 1.2 Hz, 1H), 7.05 (dd, *J* = 8.4, 1.2 Hz, 1H), 6.95 (d, J = 9.1 Hz, 2H), 4.50 (br, 1H), 4.50-4.26 (m, 2H), 3.37 (m, 5H), 3.27 (p, *J* = 6.5 Hz, 1H), 3.18 (dd, *J* = 12.4, 6.8 Hz, 1H), 1.71-1.68 (m, 6H), 1.24 (d, *J* = 4.8 Hz, 6H).

HPLC-MS: retention time= 3.09 min; m/z: 397 [M+1]⁺.

### Example I-35

### 1-(Isopropylamino)-3-(2-((4-morpholinophenyl)diazenyl)phenoxy)propan-2-ol.

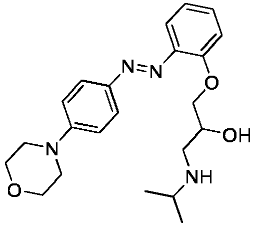

According to method L, from 39.1 mg of intermediate **(8o-6)** (0.08 mmol), 13.58 mg of compound **(I-35)** as formate salt were obtained (39% yield) once it was purified by reverse phase chromatography

1H-NMR (400 MHz, CDCl₃) δ 8.53 (br, 1H), 7.85 (d, *J =* 9.1 Hz, 2H), 7.67 (dd, *J =* 8.2, 1.6 Hz, 1H), 7.37 (ddd, *J* = 8.2, 7.2, 1.6 Hz, 1H), 7.09-7.03 (m, 2H), ), 6.97 (d, *J =* 9.1 Hz, 2H), 4.52 (br, 1H), 4.31 (dd, *J* = 9.6, 3.9 Hz, 1H), 4.24 (dd, *J* = 9.6, 5.2 Hz, 1H), 3.93-3.84 (m, 4H), 3.39 (dd, *J* = 12.4, 3.2 Hz, 5H 1H), 3.33-3.30 (m, 4H), 3.30 (h, *J =* 6.5 Hz, 1H), 3.19 (dd, *J* = 12.4, 7.5 Hz, 1H), 1.26 (d, *J =* 6.5 Hz, 6H).

HPLC-MS: retention time= 2.87 min; m/z: 399 [M+1]⁺.

### Example I-36

### 1-(isopropylamino)-3-(2-((4-(4-methylpiperazin-1-yl)phenyl)diazenyl)phenoxy) propan-2-ol (III-7)

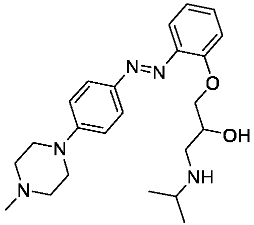

According to method L, from 38.4 mg of intermediate (8o-7) (0.08 mmol), 34.92 mg of compound (I-36) were obtained (99% yield) as trifluoroacetate salt.

¹H-NMR (400 MHz, CDCl₃) δ 7.79 (d, *J =* 9.0 Hz, 2H), 7.63 (dd, *J* = 8.3, 1.7 Hz, 1H), 7.38 (td, *J* = 8.3, 1.6 Hz, 1H), 7.06-7.02 (m, 2H), 6.98 (d, *J* = 9.0 Hz, 2H), 4.42-4.36 (m, 1H), 4.25 (dd, *J* = 9.6, 4.7 Hz, 1H), 4.17 (dd, *J* = 9.6, 5.3 Hz, 1H), 3.92-3.56 (m, 8H), 3.38-3.25 (m, 2H), 3.13 (dd, *J* = 12.5, 8.6 Hz, 1H), 2.87 (s, 3H), 1.27 (d, *J =* 6.6 Hz, 3H), 1.26 (d, *J* = 6.6 Hz, 3H).

HPLC-MS: retention time= 2.42 min; m/z: 412 [M+1]⁺; 207 [(M+2]²⁺.

### Example I-37

### 1-(isopropylamino)-3-(2-((2-methoxy-4-morpholinophenyl)diazenyl)phenoxy) propan-2-ol.

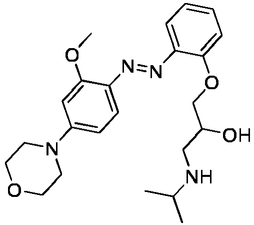

According to method L, from 4.8 mg of intermediate **(8o-9)** (0.08 mmol), 4.48 mg of compound **(I-37)** (91% yield) as trifluoroacetate salt.

¹H-NMR (400 MHz, CDCl₃) δ 7.72 (d, *J* = 8.5 Hz, 1H), 7.59 (d, *J* = 9.3 Hz, 1H), 7.34 (t, *J* = 7.5 Hz, 1H), 7.09-7.02 (m, 2H), 6.68 (dd, *J* = 9.3, 2.4 Hz, 1H), 6.48 (d, *J* = 2.4 Hz, 1H), 4.46-4.41 (m, 1H), 4.24 4.19 (m, 2H), 3.89 (t, *J* = 4.9 Hz, 4H), 3.63 (br, 4H), 3.27 (dd, *J* = 12.2, 3.2 Hz, 1H), 3.24-3.15 (m, 1H), 3.12 (dd, *J* = 12.2, 8.9 Hz, 1H), 1.33 (d, *J* = 6.2 Hz, 3H), 1.32 (d, *J* = 6.2 Hz, 3H).

HPLC-MS: retention time= 2.72 min; m/z: 429 [M+1]⁺.

### Example I-38

### 1-(Isopropylamino)-3-(2-((2-(piperidin-1-yl)phenyl)diazenyl)phenoxy)propan-2-ol.

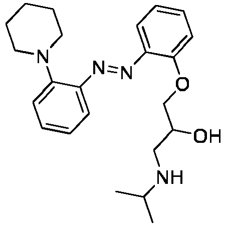

According to method L, from 15.2 mg of intermediate **(8o-11)** (0.04 mmol), 1.0 mg of compound **(I-38)** as formate salt were obtained (90% yield) once it was purified by reverse phase chromatography

HPLC-MS: retention time= 2.86 min; m/z: 397 [M+1]⁺.

### Example I-39

### 1-(2-((4-(Ethyl(2-hydroxyethyl)amino)phenyl)diazenyl)phenoxy)-3-(isopropylamino)propan-2-ol.

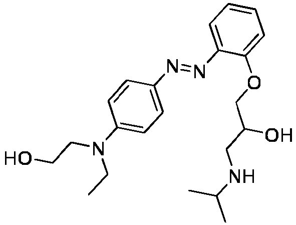

According to method L, from 15.1 mg of intermediate **(8o-14)** (0.03 mmol), 16.04 mg of compound **(I-39)** as trifluoroacetate salt were obtained (98% yield).

1H-NMR (400 MHz, CDCl₃) δ 7.84 (d, *J =* 9.3 Hz, 2H), 7.71 (dt, *J* = 8.2, 1.6 Hz, 1H), 7.34 (ddd, *J* = 8.7, 7.6, 1.6 Hz, 1H), 7.10 (t, *J* = 7.6 Hz, 1H), 7.05 (d, *J* = 8.2 Hz, 1H), 7.00 (dd, *J* = 9.3, 1.6 Hz, 2H, 2H), 4.53-4.40 (m, 1H), 4.30-4.18 (m, 2H), 3.85 (t, *J =* 6.0, 2H), 3.68 (t, *J* = 6.0 Hz, 2H), 3.63 (q, *J* = 7.2 Hz, 2H), 3.44-3.28 (m, 2H), 3.23 (dd, *J* = 12.5, 10.5 Hz, 1H), 1.32 (d, *J* = 6.8, 3H), 1.30 (d, *J* = 6.8, 3H), 1.27 (t, *J =* 7.2, 3H).

HPLC-MS: retention time= 2.88 min; m/z: 401 [M+1]⁺.

### Example I-40

### 1-(2-((4-(Diethylamino)phenyl)diazenyl)phenoxy)-3-(isopropylamino)propan-2-ol.

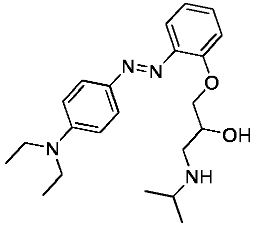

According to method L, from 19.6 mg of intermediate **(8o-15)** (0.03 mmol), 18.08 mg of compound **(I-40)** as trifluoroacetate salt were obtained (90% yield).

¹H-NMR (400 MHz, CDCl₃) δ 7.86 (d, *J* = 9.2 Hz, 2H), 7.68 (d, *J* = 8.0 Hz, 1H), 7.35 (td, *J* = 8.0, 1.6 Hz, 1H), 7.08 (dd, *J* = 8.0, 1.6 Hz, 1H) 7.07 (t, 8.0 Hz, 1H), 6.92 (d, *J* = 9.2 Hz, 2H), 4.44-4.36 (m, 1H), 4.24 (dd, *J* = 9.6, 4.8 Hz, 1H), 4.20 (dd, *J* = 9.6, 5.0 Hz, 1H), 3.53 (q, *J =* 7.1 Hz, 4H), 3.41-3.27 (m, 2H), 3.16 (dd, *J* = 12.4, 8.5 Hz, 1H), 1.31 (d, *J* = 6.6 Hz, 3H), 1.30 (d, *J =* 6.6 Hz, 3H), 1.25 (t, *J* = 7.1 Hz, 6H).

HPLC-MS: retention time= 3.19 min; m/z: 385 [M+1]⁺.

### Example I-41

### 1-(Isopropylamino)-3-(2-((2-morpholinophenyl)diazenyl)phenoxy)propan-2-ol.

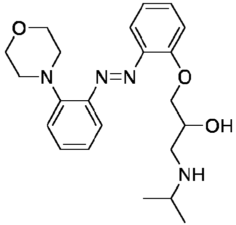

According to method L, from 19.0 mg of intermediate **(8o-19)** (0.02 mmol), 19.36 mg of compound **(I-41)** as trifluoroacetate salt were obtained (99% yield).

¹H-NMR (400 MHz, CDCl₃) δ 7.88 (dd, *J =* 7.5, 2.0 Hz, 1H), 7.58-7.49 (m, 3H), 7.31 (d, *J* = 7.8 Hz, 2H), 6.99 (t, *J* = 7.8 Hz, 1H), 6.92 (d, *J* = 8.2 Hz, 1H), 4.37-4.27 (m, 5H), 4.10 (d, *J* = 4.0 Hz, 2H), 3.43-3.29 (m, 6H), 3.21 (dd, *J =* 12.7, 3.8 Hz, 1H), 1.37 (d, *J* = 6.6, 3H), 1.35 (d, *J =* 6.6, 3H).

HPLC-MS: retention time= 2.82 min; m/z: 399 [M+1]⁺.

### Photoisomerization

All samples were prepared at 30 µM of compound in 0.5 % DMSO in aqueous buffer (PBS, Phosphate-buffered saline) in 96-well transparent plates (200 µL of compound solution/well) at 25 °C. Absorbance at 405 nm was recorded first in darkness using a Tecan Spark microplate reader. Samples were illuminated for 3 minutes at 405 nm using a Teleopto LED array system.

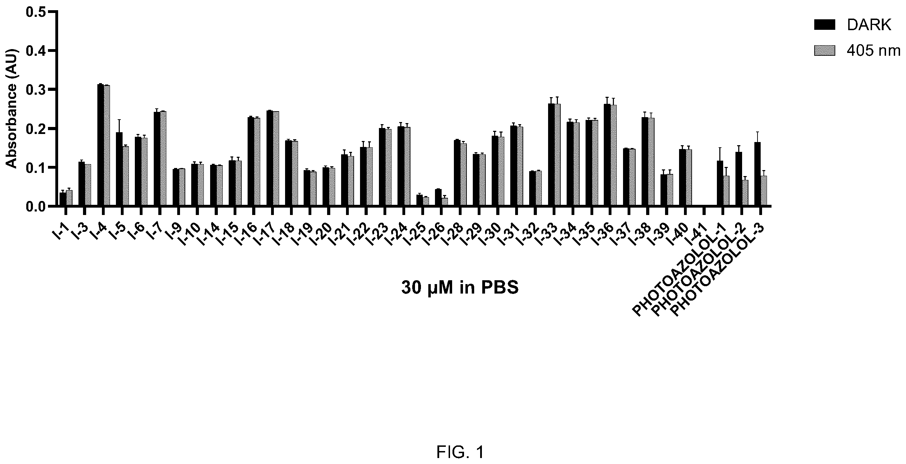

Photoazolol-1, Photoazolol-2 and Photoazolol-3 are used as standards and were described in Duran-Corbera A et al. (J. Med. Chem. 13;63(15):8458-8470). A representation of the maximum absorbance at 405 nm of each compound in light and dark condition is shown in Figure 1.

### Thermal relaxation time

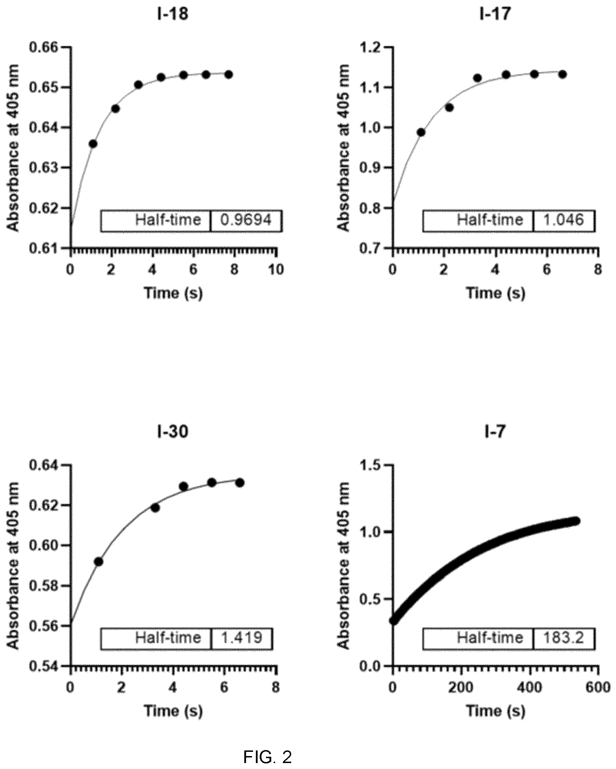

To determine the thermal relaxation time in which the metastable isomer reverts to the thermodynamically stable isomer (cis-azobenzene to trans-azobenzene), 200 µL of compound at 30 µM in 0.5 % DMSO in aqueous buffer (PBS) at 25 °C was dispensed in a Hellma QS cuvette. The cuvette was placed in the autosampler of a Thermo UV-Vis spectrophotometer and irradiated for 3 min at 405 nm with a CoolLED system. Immediately right after switch off the light source, the absorbance was kinetically recorded at 405 nm using spectrometer (bandwidth 2 nm, integration 1 sec, measurement every 1 sec). The absorbance value is plotted against time on a graph (Figure 2) and the thermal relaxation half-time is calculated by non-linear regression in GraphPad software, using the equation: $Y = Y 0 + \left(Plateau-Y0\right) * \left(1-exp\left(-K*x\right)\right)$

Where *Y0* is the *Y* value when *X* (time) is zero. Plateau is the Y value at infinite times. K is the rate constant, expressed in reciprocal of the *X* axis time units. If *X* is in seconds, then *K* is expressed in inverse seconds. The half-time is in the time units of the *X* axis, computed as *In(2)*/*K.* Table 1 shows the half-time obtained for some compounds of the invention.

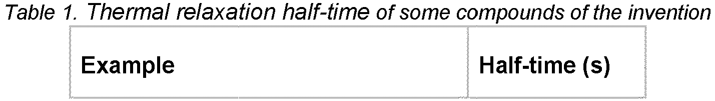

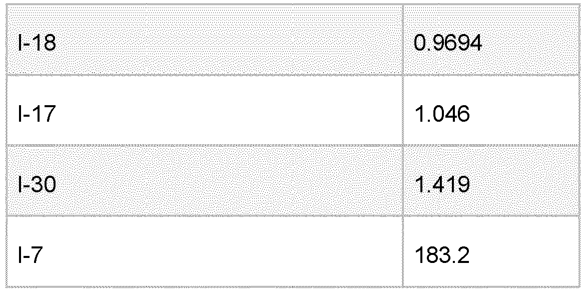

### Cell culture assays

The activity of photoswitchable ligands against β2-AR was evaluated using HEK293 M1 cells, which stably express a cAMP Epac FRET biosensor and have endogenous β2-AR.

On the other hand, to evaluate the functional activity of β1-AR, the iSNAP β1-AR HEK-293 polyclonal cell line was used, which can express β1-AR upon induction with doxycycline and also contain the cAMP Epac FRET biosensor.

100,000 cells per well were seeded in a transparent 96-well microplate (Thermo Scientific Nunc Microwell) and left at 37°C with 5% CO2 overnight. In the case of assays performed using iSNAP β1-AR HEK-293, the receptor expression was induced by addition of 0.01 µg/mL doxycycline to the medium 24 h. The cAMP EPAC sensor buffer (14 mM NaCl, 50 nM KCI, 10 nM MgCl2, 10 nM CaCl2, 1 mM HEPES, 1.82 mg/mL Glucose, pH 7.2) was used as the assay medium in all the experiments. In assays performed using the HEK293 M1 cell line, cAMP EPAC sensor buffer was supplemented with 100 µM IBMX to inhibit endogenous phosphodiesterases. Fluorescence values were measured using a Tecan Spark microplate reader with defined wavelength settings (excitation filter 430/20 nm and emission filters 485/20 nm and 535/25 nm). FRET ratio was calculated as the ratio of the donor emission (485 nm) divided by the acceptor emission (535 nm). The FRET ratio was normalized to the effect of the buffer (0%) and the maximum response obtained with control compounds (100%). Each set of experiments was performed three to five times with each concentration in duplicate. The plate was incubated with the studied compounds for 45 minutes in the dark at room temperature. Fluorescence values were thereafter measured. To induce photoswitching, the plate was exposed to continuous illumination at 405 nm for 15 min using the LED array plate (LEDA Teleopto). Fluorescence values were measured again. The data of of beta-adrenergic compounds (β1) under illumination is shown in Table 2, whereas pharmacological data of beta-adrenergic compounds (β₂) under illumination is shown in Table 3.

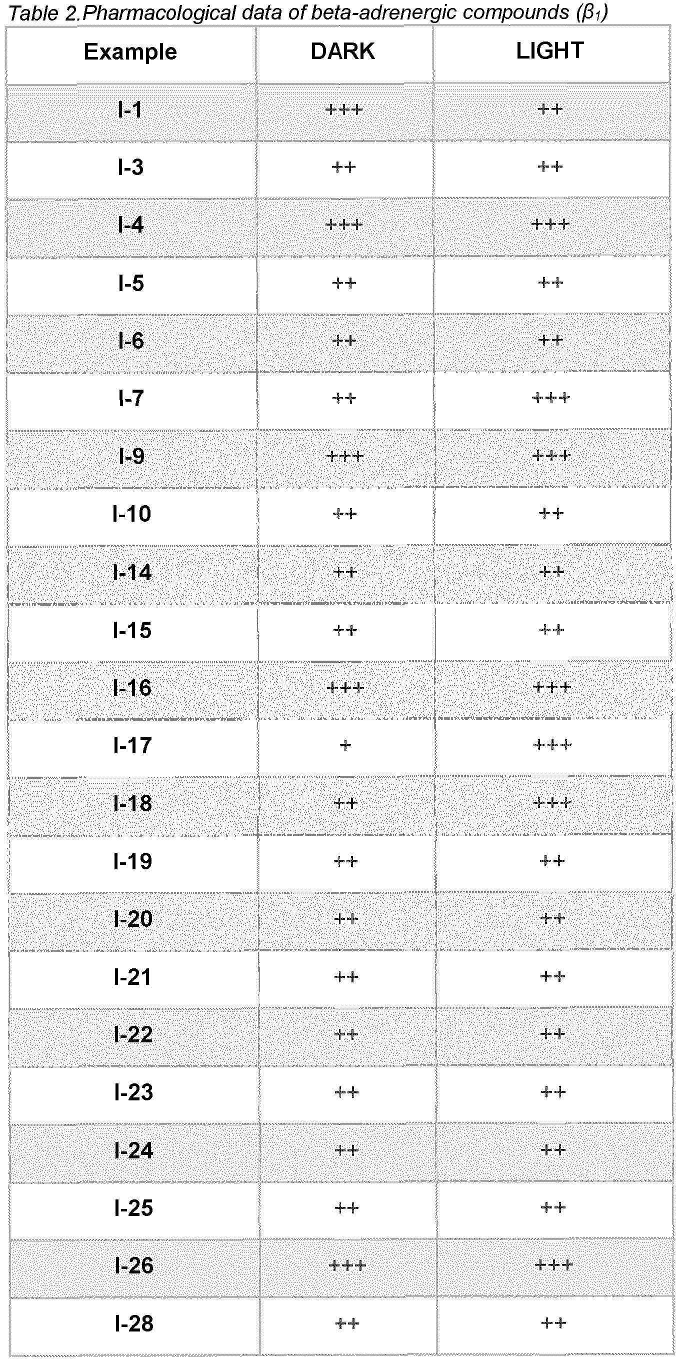

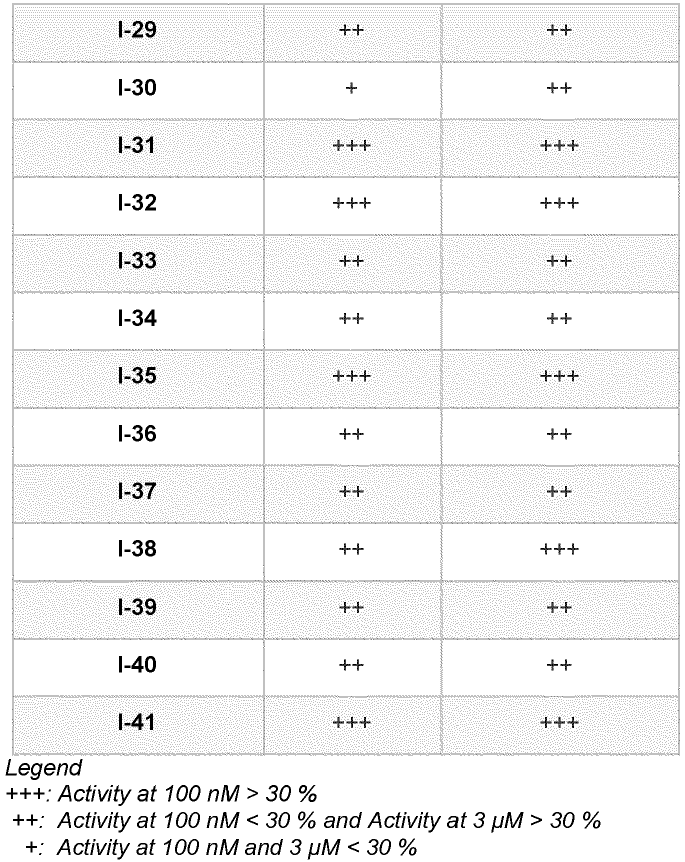

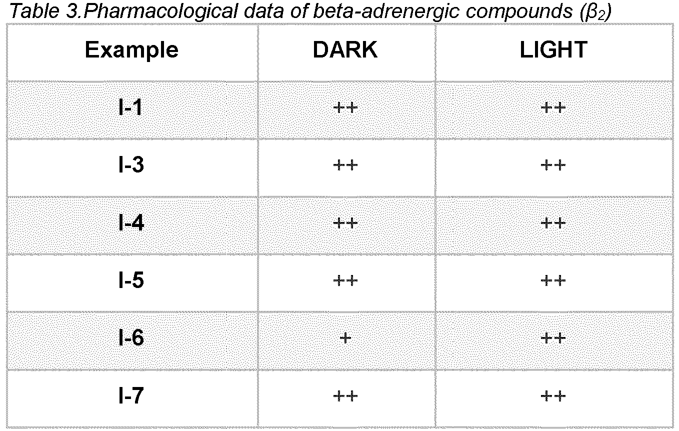

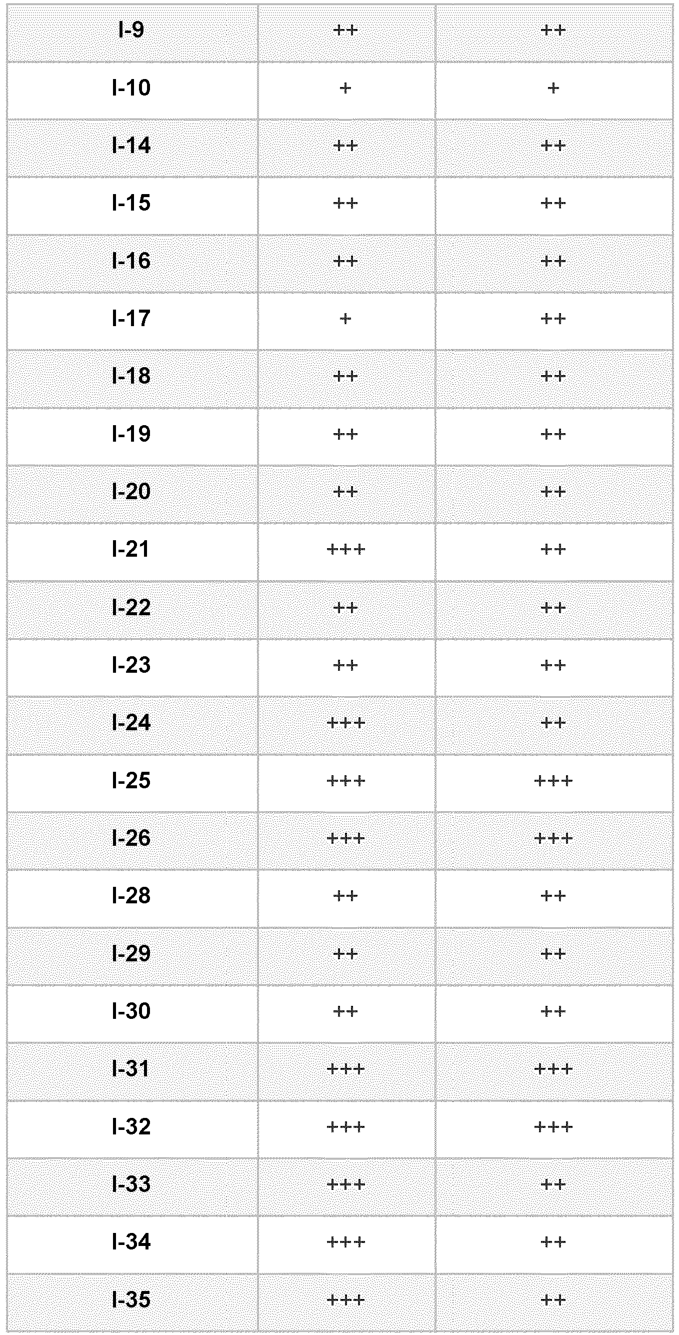

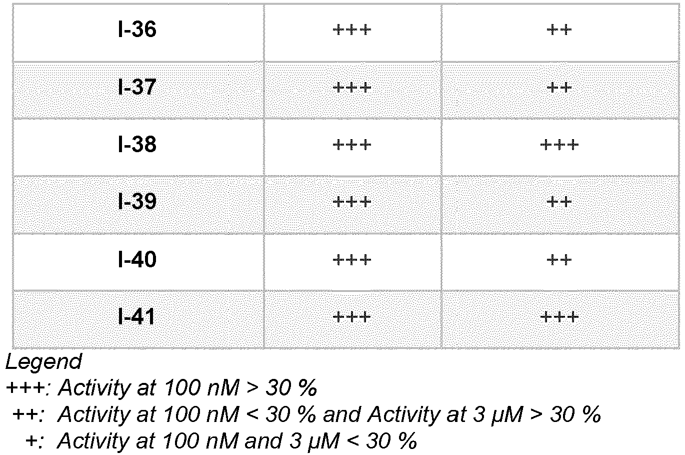

### In vivo intraocular pressure (IOP) assays

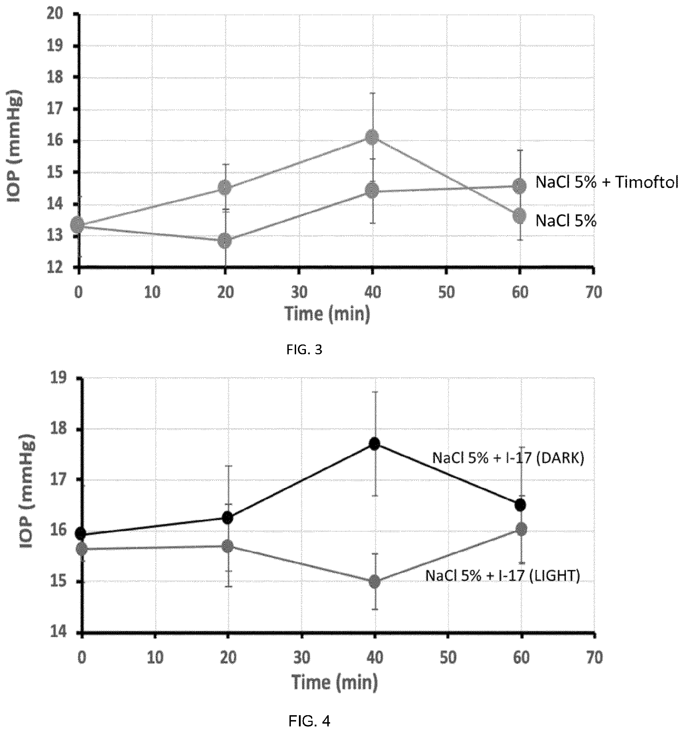

Intraocular pressure was evaluated in pigmented C57BL6J mice (>2 months old) subjected to an experimental protocol to increase in IOP (bilateral intraocular injection of 2 µL of 5% NaCl solution). Measurements were obtained using digital tonometry (Tono-Lab^{®}) before the injection and at 20, 40, and 60 minutes post-injection across four experimental groups: Group 1 (Control) received only 5% NaCl; Group 2 was treated with Timoftol^{®} (Gold Standard) applied topically immediately after NaCl injection (Figure3); Group 3 received compound I-17 (30 µM) topically after NaCl injection without light stimulation; and Group 4 received compound 1-17 (30 µM) topically after NaCl injection with monochromatic light stimulation at 470 nm (Figure 4). Timoftol^{®} is a medicament containing 5 mg/ml of beta blocker timolol and commercialized in Spain by Santen Oy.

## Claims

1. Compound of formula (I): 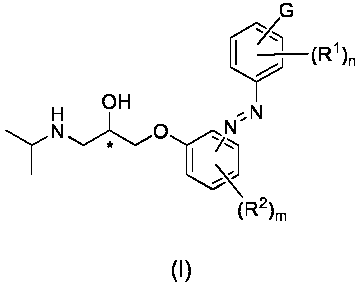
or a salt, solvate or stereoisomer thereof,
wherein
n is selected from 0, 1 and 2;
G is a nitrogen containing group selected from the group consisting of linear or branched amine and nitrogen containing heterocyclic ring, wherein said group is attached to the phenyl ring through a nitrogen atom;
wherein the nitrogen containing heterocyclic ring is optionally substituted with a substituent selected from the group of linear or branched C₁₋₄ alkyl, C₁₋₄ alkoxy, OH, CN and halogen
R¹ is selected from the group consisting of linear or branched C₁-C₆ alkyl optionally substituted with a OH group, OR³group, halogen, OCF₃, CF₃, CN, NH₂, and NO₂;
wherein R³ is selected from linear or branched C₁-C₃ alkyl;
m is selected from 0, 1 and 2; and
R² is selected from the group consisting of linear or branched C₁-C₆ alkyl optionally substituted with a OH group, OR⁴ group, halogen, OCF₃, CF₃, CN, NH₂, and NO₂;
wherein R⁴ is selected from linear or branched C₁-C₃ alkyl.

2. Compound according to claim 1, wherein
n is selected from 0, 1 and 2;
G is selected from the group consisting of N(C₁-C₆ alkyl)₂, NH₂, NH(C₁-C₆ alkyl), N(C₁-C₆ alkyl)(C₁-C₆ alkyl-OH) and nitrogen containing heterocyclic ring;
wherein the C₁-C₆ alkyl is selected from linear or branched C₁-C₆ alkyl optionally substituted;
wherein the nitrogen containing heterocyclic ring is optionally substituted with a substituent selected from the group of linear or branched C₁₋₄ alkyl, C₁₋₄ alkoxy, OH, CN and halogen;
R' is selected from linear or branched C₁-C₆ alkyl optionally substituted with a OH group, and a OR³ group;
wherein R³ is selected from linear or branched C₁-C₃ alkyl
m is selected from 0, 1 and 2; and
R² is selected from the group consisting of linear or branched C₁-C₆ alkyl optionally substituted with a OH group and a OR⁴group;
wherein R⁴ is selected from linear or branched C₁-C₃ alkyl.

3. Compound according to claim 1 or 2, wherein
n is selected from 0, and 1;
G is selected from the group consisting of N(C₁-C₃ alkyl)₂, NH₂; NH(C₁-C₃ alkyl), -N(C₁-C₃ alkyl)(C₁-C₃ alkyl-OH) and nitrogen containing heterocyclic ring;
wherein the C₁-C₃ alkyl is selected from linear or branched C₁-C₃ alkyl optionally substituted;
wherein the nitrogen containing heterocyclic ring is optionally substituted with a substituent selected from the group of linear or branched C₁₋₄ alkyl, C₁₋₄ alkoxy, OH, CN and halogen;
R' is linear or branched C₁-C₃ alkyl or a OCH₃ group;
m is selected from 0 and 1; and
R² is linear or branched C₁-C₃ alkyl or a OCH₃ group.

4. Compound according to any of the precedent claims, wherein
n is selected from 0, and 1;
G is selected from the group consisting of N(C₁-C₂ alkyl)₂; NH₂; NH(C₁-C₂ alkyl) and N(C₁-C₂ alkyl)(C₁-C₂ alkyl-OH);
wherein the C₁-C₂ alkyl is selected from C₁-C₂ alkyl optionally substituted;
R¹ is linear or branched C₁-C₃ alkyl or a OCH₃ group;
m is selected from 0 and 1; and
R² is linear or branched C₁-C₃ alkyl or a OCH₃ group.

5. Compound according to any of the precedent claims, wherein m is 0.

6. Compound according to any of the precedent claims, wherein the diazo group is located in meta position with respect to the 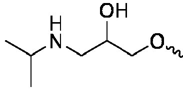 group, and
wherein the wavy line represents the point of attachment to the rest of the molecule.

7. Compound according to any of the precedent claims, wherein the G group is located in ortho or para position with respect to the diazo group.

8. Compound according to any of the precedent claims, wherein R¹ is located in ortho or meta position with respect to the diazo group.

9. Compound according to claims 1 to 3, selected from the group consisting of:
(S)-1-(4-((4-(Dimethylamino)phenyl)diazenyl)phenoxy)-3-(isopropylamino) propan-2-ol,
(R)-1-(4-((4-(Dimethylamino)phenyl)diazenyl)phenoxy)-3-(isopropylamino) propan-2-ol,
1-(Isopropylamino)-3-(4-((4-(methylamino)phenyl)diazenyl)phenoxy)propan-2-ol,
1-(4-((4-(Azetidin-1-yl)phenyl)diazenyl)phenoxy)-3-(isopropylamino)propan-2-ol,
1-(Isopropylamino)-3-(4-((4-(pyrrolidin-1-yl)phenyl)diazenyl)phenoxy)propan-2-ol,
1-(Isopropylamino)-3-(4-((4-(piperidin-1-yl)phenyl)diazenyl)phenoxy)propan-2-ol,
1-(Isopropylamino)-3-(4-((4-morpholinophenyl)diazenyl)phenoxy)propan-2-ol,
1-(isopropylamino)-3-(4-((4-(4-methylpiperazin-1-yl)phenyl)diazenyl)phenoxy ) propan-2-ol,
1-(4-((4-(Dimethylamino)-2-methoxyphenyl)diazenyl)phenoxy)-3-(isopropyla mino)propan-2-ol,
1-(Isopropylamino)-3-(4-((2-methoxy-4-morpholinophenyl)diazenyl)phenoxy) propan-2-ol
1-(4-((2-(Dimethylamino)phenyl)diazenyl)phenoxy)-3-(isopropylamino)propan -2-ol,
1-(Isopropylamino)-3-(4-((2-(piperidin-1-yl)phenyl)diazenyl)phenoxy)propan-2-ol,
1-(Isopropylamino)-3-(4-((2-morpholinophenyl)diazenyl)phenoxy)propan-2-ol,
1-(4-((4-Aminophenyl)diazenyl)phenoxy)-3-(isopropylamino)propan-2-ol,
1-(4-((4-(Ethyl(2-hydroxyethyl)amino)phenyl)diazenyl)phenoxy)-3-(isopropyla mino)propan-2-ol,
1-(4-((4-(Diethylamino)phenyl)diazenyl)phenoxy)-3-(isopropylamino)propan-2-ol,
(S)-1-(3-((4-(Dimethylamino)phenyl)diazenyl)phenoxy)-3-(isopropylamino)pro pan-2-ol,
1-(Isopropylamino)-3-(3-((4-(methylamino)phenyl)diazenyl)phenoxy)propan-2-ol,
1-(3-((4-(Azetidin-1-yl)phenyl)diazenyl)phenoxy)-3-(isopropylamino)propan-2-ol,
1-(Isopropylamino)-3-(3-((4-(pyrrolidin-1-yl)phenyl)diazenyl)phenoxy)propan-2-ol,
1-(Isopropylamino)-3-(3-((4-(piperidin-1-yl)phenyl)diazenyl)phenoxy)propan-2-ol,
1-(Isopropylamino)-3-(3-((4-morpholinophenyl)diazenyl)phenoxy)propan-2-ol,
1-(Isopropylamino)-3-(3-((4-(4-methylpiperazin-1-yl)phenyl)diazenyl)phenoxy ) propan-2-ol,
1-(isopropylamino)-3-(3-((2-methoxy-4-morpholinophenyl)diazenyl)phenoxy) propan-2-ol,
1-(3-((2-(Dimethylamino)phenyl)diazenyl)phenoxy)-3-(isopropylamino)propan -2-ol,
1-(Isopropylamino)-3-(3-((2-(piperidin-1-yl)phenyl)diazenyl)phenoxy)propan-2-ol,
1-(Isopropylamino)-3-(3-((2-(morpholinophenyl)diazenyl)phenoxy)propan-2-ol
1-(3-((4-Aminophenyl)diazenyl)phenoxy)-3-(isopropylamino)propan-2-ol,
1-(3-((4-(Ethyl(2-hydroxyethyl)amino)phenyl)diazenyl)phenoxy)-3-(isopropyla mino)propan-2-ol,
1-(3-((4-(Diethylamino)phenyl)diazenyl)phenoxy)-3-(isopropylamino)propan-2-ol,
(S)-1-(2-((4-(Dimethylamino)phenyl)diazenyl)phenoxy)-3-(isopropylamino)pro pan-2-ol,
1-(Isopropylamino)-3-(2-((4-(methylamino)phenyl)diazenyl)phenoxy)propan-2-ol,
1-(2-((4-(azetidin-1-yl)phenyl)diazenyl)phenoxy)-3-(isopropylamino)propan-2-ol,
1-(Isopropylamino)-3-(2-((4-(piperidin-1-yl)phenyl)diazenyl)phenoxy)propan-2-ol,
1-(Isopropylamino)-3-(2-((4-morpholinophenyl)diazenyl)phenoxy)propan-2-ol,
1-(isopropylamino)-3-(2-((4-(4-methylpiperazin-1-yl)phenyl)diazenyl)phenoxy) propan-2-ol,
1-(isopropylamino)-3-(2-((2-methoxy-4-morpholinophenyl)diazenyl)phenoxy)pr opan-2-ol,
1-(Isopropylamino)-3-(2-((2-(piperidin-1-yl)phenyl)diazenyl)phenoxy)propan-2-ol,
1-(2-((4-(Ethyl(2-hydroxyethyl)amino)phenyl)diazenyl)phenoxy)-3-(isopropyla mino)propan-2-ol,
1-(2-((4-(Diethylamino)phenyl)diazenyl)phenoxy)-3-(isopropylamino)propan-2-ol, and
1-(Isopropylamino)-3-(2-((2-morpholinophenyl)diazenyl)phenoxy)propan-2-ol.

10. Compound according to claim 9, selected from the group consisting of:
(S)-1-(3-((4-(Dimethylamino)phenyl)diazenyl)phenoxy)-3-(isopropylamino)pro pan-2-ol;
1-(Isopropylamino)-3-(3-((4-(methylamino)phenyl)diazenyl)phenoxy)propan-2-ol;
1-(3-((4-(Azetidin-1-yl)phenyl)diazenyl)phenoxy)-3-(isopropylamino)propan-2-ol;
1-(Isopropylamino)-3-(3-((4-(pyrrolidin-1-yl)phenyl)diazenyl)phenoxy)propan-2-ol;
1-(Isopropylamino)-3-(3-((4-(piperidin-1-yl)phenyl)diazenyl)phenoxy)propan-2-ol;
1-(Isopropylamino)-3-(3-((4-morpholinophenyl)diazenyl)phenoxy)propan-2-ol;
1-(Isopropylamino)-3-(3-((4-(4-methylpiperazin-1-yl)phenyl)diazenyl)phenoxy ) propan-2-ol;
1-(isopropylamino)-3-(3-((2-methoxy-4-morpholinophenyl)diazenyl)phenoxy) propan-2-ol;
1-(3-((2-(Dimethylamino)phenyl)diazenyl)phenoxy)-3-(isopropylamino)propan -2-ol;
1-(Isopropylamino)-3-(3-((2-(piperidin-1-yl)phenyl)diazenyl)phenoxy)propan-2-ol;
1-(Isopropylamino)-3-(3-((2-morpholinophenyl)diazenyl)phenoxy)propan-2-ol;
1-(3-((4-Aminophenyl)diazenyl)phenoxy)-3-(isopropylamino)propan-2-ol;
1-(3-((4-(Ethyl(2-hydroxyethyl)amino)phenyl)diazenyl)phenoxy)-3-(isopropyla mino)propan-2-ol; and
1-(3-((4-(Diethylamino)phenyl)diazenyl)phenoxy)-3-(isopropylamino)propan-2-ol.

11. Compound according to claim 10 selected from the group consisting of (S)-1-(3-((4-(Dimethylamino)phenyl)diazenyl)phenoxy)-3-(isopropylamino)propan-2-ol;
1-(Isopropylamino)-3-(3-((4-(methylamino)phenyl)diazenyl)phenoxy)propan-2-ol; and
1-(3-((4-(Diethylamino)phenyl)diazenyl)phenoxy)-3-(isopropylamino)propan-2-ol.

12. Pharmaceutical composition comprising a compound of formula (I) as defined in claims 1-11, or a salt, solvate or stereoisomer thereof, and at least one pharmaceutically acceptable excipient.

13. Compound according to any one of claims 1 to 11 for use as a medicament.

14. Compound according to any one of claims 1 to 11 for use in the treatment of an ocular disease.

15. Compound according to claim 14, wherein the ocular disease is selected from the group consisting of glaucoma, ocular hypertension and diabetic corneal sensory nerve dysfunction, preferably glaucoma.
